# EUROPEAN PATENT SPECIFICATION

(11) **EP 3 442 594 B1**
(45) Date of publication and mention of the grant of the patent: **07.02.2024**
(21) Application number: 17721952.4
(22) Date of filing: 10.04.2017
(51) Int. Cl.: A61K 31/00, A61K 47/60, A61K 47/58, A61K 47/65, A61K 47/69, A61K 9/107, A61P 35/00, A61P 35/02, A61K 31/337, A61K 31/704, A61K 31/407

(54) **BLOCK COPOLYMER FOR OVERCOMING DRUG RESISTANCE OF TUMOURS TO CHEMOTHERAPY, ITS POLYMER-DRUG CONJUGATE, PHARMACEUTICAL COMPOSITION CONTAINING THEM, METHOD OF PREPARATION AND USE THEREOF**
BLOCKCOPOLYMER ZUR ÜBERWINDUNG DER ARZNEIMITTELRESISTENZ VON TUMOREN GEGEN CHEMOTHERAPIE, DESSEN POLYMER-WIRKSTOFF-KONJUGAT, PHARMAZEUTISCHE ZUSAMMENSETZUNG DAMIT, VERFAHREN ZUR HERSTELLUNG UND VERWENDUNG DAVON
COPOLYMÈRE SÉQUENCÉ POUR SURMONTER LA PHARMACORÉSISTANCE DE TUMEURS À UNE CHIMIOTHÉRAPIE, SON CONJUGUÉ POLYMÈRE-MÉDICAMENT, COMPOSITION PHARMACEUTIQUE CONTENANT CELUI-CI, PROCÉDÉ DE PRÉPARATION ET UTILISATION DE CELUI-CI

(30) Priority: 11.04.2016 CZ 20160206
(43) Date of publication of application: 20.02.2019
(73) Proprietor: Ustav makromolekularni chemie AV CR, v.v.i., 16206 Praha 6 - Brevnov (CZ); Mikrobiologicky ustav AV CR, v.v.i., 14220 Praha 4 (CZ)
(72) Inventor: ETRYCH, Tomas, 25210 Mnisek pod Brdy (CZ); KOSTKA, Libor, 27054 Revnicov (CZ); BRAUNOVA, Alena, 10100 Praha 10 (CZ); CUCHALOVA, Lucie, 13000 Praha 3 (CZ); SIROVA, Milada, 14000 Praha 4 (CZ); JANOUSKOVA, Olga, 25001 Brandýs nad Labem (CZ)
(74) Representative: Hartvichova, Katerina
(86) International application number: PCT/CZ2017/050015
(87) International publication number: WO 2017/177991

(56) References cited:
- WO-A1-2008/034391
- DARIA Y. ALAKHOVA ET AL: "Pluronics and MDR Reversal: An Update", MOLECULAR PHARMACEUTICS, vol. 11, no. 8, 4 August 2014 (2014-08-04) , pages 2566-2578, XP055390943, US ISSN: 1543-8384, DOI: 10.1021/mp500298q
- BRAUNOVÁ ALENA ET AL: "Tumor-targeted micelle-forming block copolymers for overcoming of multidrug resistance", JOURNAL OF CONTROLLED RELEASE, vol. 245, 18 November 2016 (2016-11-18), pages 41-51, XP029877443, ISSN: 0168-3659, DOI: 10.1016/J.JCONREL.2016.11.020
- Braunová Alena ET AL: "BLOCK COPOLYMERS OF POLY(N-2-HYDROXYPROPYL METHACRYLAMIDE) AND POLY(PROPYLENE GLYCOL) - THE WAY TO INHIBIT P-GLYCOPROTEIN?", , 16 March 2015 (2015-03-16), XP055635761, Retrieved from the Internet: URL:https://novel-drugdelivery-systems.pha rmaceuticalconferences.com/speaker/2015/al ena-braunova-institute-of-macromolecular-c hemistry-as-cr-czech-republic [retrieved on 2019-10-24]
- LARSON N ET AL: "Polymeric Conjugates for Drug Delivery", CHEMISTRY OF MATERIALS, vol. 24, no. 5, 13 March 2012 (2012-03-13) , pages 840-853, XP055103531, ISSN: 0897-4756, DOI: 10.1021/cm2031569
- PISZKIEWICZ D ET AL: "Anomalous cleavage of aspartyl-proline peptide bonds during amino acid sequence determinations", BIOCHEMICAL AND BIOPHYSICAL RESEARCH COMMUNICATIONS, ELSEVIER, AMSTERDAM NL, vol. 40, no. 5, 10 September 1970 (1970-09-10), pages 1173-1178, XP024847889, ISSN: 0006-291X, DOI: 10.1016/0006-291X(70)90918-6 [retrieved on 1970-09-10]
- PECHAR M: "BIODEGRADABLE DRUG CARRIERS BASED ON POLY(ETHYLENE GLYCOL) BLOCK COPOLYMERS", MACROMOLECULAR CHEMISTRY AND PHYSICS, WILEY-VCH VERLAG, WEINHEIM, DE, vol. 198, no. 4, 1 April 1997 (1997-04-01) , pages 1009-1020, XP000677485, ISSN: 1022-1352, DOI: 10.1002/MACP.1997.021980408
- ZHONG YJ ET AL: "Cathepsin B-cleavable doxorubicin prodrugs for targeted cancer therapy (Review)", INTERNATIONAL JOURNAL OF ONCOLOGY, 28 December 2012 (2012-12-28), XP055207310, ISSN: 1019-6439, DOI: 10.3892/ijo.2012.1754

## Description

### Field of Art

The invention relates to a block copolymer for overcoming drug resistance of tumours to chemotherapy, its polymer-drug conjugate, pharmaceutical compositions containing them, methods for their preparation and their use as micellar drug carriers allowing overcoming multiple drug resistance of tumours.

### Background Art

Currently, in the treatment of many diseases, especially cancer, there are efforts to utilize more fully the polymer-based substances which may serve as the so-called polymer drug carriers. The condition of their use for medical purposes is their biocompatibility, ability to reduce non-specific toxicity of a bound drug compared to a low molecular weight drug, prolonged circulation of the drug in the bloodstream, the possibility of elimination of the polymer carrier from the body (preferably by glomerular filtration) after delivery the drug to its targetand, finally, drug accumulation in the target tissue and its controlled release from the polymer carrier. It has been previously found that the high molecular weight of polymer carriers and their conjugates with drugs significantly influences and contributes to the so-called passive drug accumulation in solid tumours. This phenomenon is called the enhanced permeability and retention (EPR) effect. On the basis thereof, in solid tumours there are preferentially accumulated high molecular weight substances both water-soluble and those forming particles in water with sizes of several tens to hundreds of nanometres. This fact is now extensively used for the preparation of polymer conjugates with a covalently bound cancerostatic, specifically accumulating in the majority of solid tumours. A low molecular weight cancerostatic agent is bound to the polymer carrier via a degradable bond allowing controlled release of the delivered drug from the carrier in its original active form either in the tumour tissue, or directly in the tumour cell.

Recent years have seen a sharp increase in the research and development of polymer drug carriers based on copolymers of N-(2-hydroxypropyl)methacrylamide (pHPMA), which are water-soluble, non-toxic to living organisms, entirely biocompatible with living tissues and also allow binding of e.g. drug, ABC transporters inhibitor, structures enabling targeting towards cell receptors, and other biologically active molecules.

PHPMAs allow modification of the main chain by another polymer chain, both at chain ends and along the chain by the so called grafting. Other types of polymers studied and utilized in clinical practice as various types and forms of polymer drug carriers are e.g. various derivatives of poly(ethylene oxide), copolymers of poly(ethylene oxide) and poly(propylene oxide) (Pluronic or Poloxamer), poly(A-vinylpyrrolidone), copolymers of lactic and glycolic acids, derivatives of polyphosphazenes, polyanhydrides, poly(ε-caprolactone), polyphosphoesters, poly(cyanoacrylate), poly(methyl acrylate)s and poly(methacrylic acid), poly(N-isopropylacrylamide) and others. Binding a low molecular weight cancerostatic drug to polymer carrier via a covalent bond utilizes biodegradable linkages, e.g. amide, hydrazone, acetal, disulphide (-S-S-) and the like. These linkages are usually stable in the bloodstream and are cleaved specifically in the environment of the tumour or tumour cells. Therefore, there is no risk that the low molecular weight cancerostatic releases non-specifically already during circulation in the bloodstream, where it would act toxically and moreover, it would be eliminated from the organism in a very short time without reaching the desired target (e.g. a tumour).

Delivery of a cancerostatic agent to the place of desired effect is one, but not the only prerequisite for successful treatment of cancer. Repeated cytostatic treatment often reduces its effectiveness due to resistance of tumour cells to structurally and functionally different cytotoxic drugs, in particular anthracycline antibiotics, including e.g. doxorubicin (DOX). The resistance of tumour cells to drugs (the multidrug resistance, MDR) is usually caused by prolonged exposure of these cells to cytotoxic agents and so their defensive reaction is to reduce the intracellular concentration of the drug by various mechanisms. One of the most significant and therefore also frequently studied mechanisms is the drug efflux which involves using special transmembrane proteins to "pump" cytostatic agents out of the cell before they can act inside the cell. In most cases, this phenomenon is caused by a high molecular weight membrane glycoprotein called P-glycoprotein (P-gp). P-gp belongs to the superfamily of ABC transporters, which are characterized by two ATP domains where ATP hydrolysis takes place with release of energy required to transport the substrate out of the cell. P-gp is an ATP-dependent efflux pump for endogenous or exogenous substances with a broad substrate specificity which clearly distinguishes it from other ABC transporters whose substrates are narrow groups of substances. P-glycoprotein is a part of the plasma membrane of cells and is located in the cells of many important organs such as the liver, kidney, intestines, and others where it participates in the active transport of ions, amino acids, peptides, sugars, but also drugs and their metabolites from inside the cells to extracellular environment. While in healthy cells P-gp acts as a protection against penetration of xenobiotics in the body, in the case of tumour cells wherein the protein is expressed in much larger quantities it contributes thanks to this property to the resistance of cancer cells to the effects of drugs. Through inhibition of this process it is possible to achieve better penetration of drugs into cells and maintain here a sufficiently high level of the drug required for effective therapy.

One of the possible solutions how to achieve inhibition of the P-gp function, and thereby permit the entry of the drug into cell and its activity inside, is a structural modification of drugs or use of special P-gp inhibitors in combination with a therapeutic agent. Using the inhibitor alone, however, has very serious side effects. Moreover, the threat of inhibition of the P-gp function in normal, thus healthy cells is very real. At present, scientists are trying to address the issue of side effects of P-gp inhibitors (e.g. ritonavir- or reversin-based) by their binding to a macromolecular carrier to which also a cytostatic agent is bound, or by combining a polymer inhibitor with a polymer cancerostatic.

Another alternative is the use of a polymer-drug conjugate in which part of the polymer carrier also acts as a P-gp inhibitor. From literature there are known studies of Pluronic-based micellar carriers, triblock copolymer of poly(ethylene oxide) (PEO) and poly(propylene oxide) (PPO), PEO-PPO-PEO, which has been found to be capable to inihibit P-gp. Micellar systems based on Pluronic can be used as solubilizers of water-insoluble drugs (such as DOX) and as "nanocontainers" for these drugs which are to be released only at the specific site of action (e.g. in a solid tumour). Polymer micelles, polymersomes or other forms of nanoparticles such as dendrimer-based nanosystems are a type of polymer carrier, which should be able (due to the EPR effect) to passively target the whole system to vascularized solid tumours, where the increased accumulation of a high molecular medicine should occur. Micelles consist of a hydrophobic core in which a low molecular drug is incorporated either by physical interactions or covalently and a hydrophilic shell which is mostly composed of hydrophilic polymers (usually based on PEO) protecting the entire system from aggregation and from interacting with the components of live organism (macrophages and cells of the immune system, RES cells) and trapping in the liver. Systems based on high-molecular weight substances, whether in the form of water-soluble carriers, micelles, liposomes, polymersomes or other nanosystems, promise more efficient and safer use of cancerostatics compared to the current classical chemotherapy.

Drugs which bypass the multiple drug resistance by inhibiting the function of P-glycoprotein (P-gp) by PEO and PPO block copolymers (US 20130195964 (A1); Batrakova, E.V.; Kabanov, A.V., J. Control. Release 2008; 130: 988-106; Chen, L.; Sha, X.; Jiang, X.; Chen, Y.; Ren, Q.; Fang, X., Int. J. Nanomedicine 2013; 8: 73-84; Alakhova, D.Y.; Rapoport, N.Y.; Batrakova, E.V.; Timoshin, A.A.; Li, S.; Nicholls, D.; Alakhov, V.Y., Kabanov, A.V.; J. Control. Release 2010; 142(1): 89-100; Alakhova, D.Y.; Zhao, Y.; Li, S.; Kabanov, A.V., PLOS ONE, 2013; 8(8): e72238) and which also use these amphiphilic copolymers as micellar drug carriers, have a hydrophobic drug trapped within the hydrophobic core of the micelle only by hydrophobic interactions, and it is released by a combination of diffusion and disintegration of micelle when the concentration of the copolymer in body fluids is decreased below the critical micelle concentration (CMC). The disadvantage of systems with non-covalently incorporated drugs is the permanent release of the drug due to diffusion, even in healthy parts of the organism, during transport of the system to the target tissue.

At the 5th International Conference and Exhibition on Pharmaceutics & Novel Drug Delivery Systems in March 2015 in Dubai Braunová et al. presented a drug conjugate comprising a block copolymer of pHPMA and PPO wherein pHPMA and PPO are covalently linked for overcoming drug resistance of tumours to chemotherapy.

### Disclosure of the Invention

The present invention relates to a block copolymer, its biodegradable conjugate with a low molecular weight drug, and compositions for the treatment especially of cancer. The resulting polymer cancerostatics enable a targeted therapy particularly of poorly treatable resistant tumour diseases in human medicine. These polymer cancerostatics allow for a targeted effect of cancerostatics, in particular doxorubicin, on solid tumours while overcoming multiple drug resistance (MDR) of tumours to chemotherapy. The invention relates to an amphiphilic block copolymer, or its conjugate with a drug, which in an aqueous medium forms polymer micelles whose size and structure are controlled by the hydrophilicity and hydrophobicity of each block of the block copolymer, their length and the ratio of the hydrophilic and hydrophobic parts of the molecule. Polymer micelles, which are also an object of the present invention, comprise a hydrophilic shell consisting of a hydrophilic polymer based on N-(2-hydroxypropyl)methacrylamide (HPMA) and a hydrophobic core formed by a polymer based on poly(propylene oxide) (PPO). Both polymers are bound to one another via a biodegradable spacer that permits disintegration of the block copolymer into individual blocks upon reaching the conditions of biodegradation. A relevant drug is bound to the polymer by a pH-sensitive covalent bond which prevents accidental release of the drug in body fluids through diffusion or disintegration of micelles when the concentration of the copolymer is reduced below the critical micelle concentration (CMC), and the drug is thus released from the block copolymer only after pH of the surrounding tissue has changed, which is typically in the target tumour tissue. Moreover, the block copolymer of the present invention itself acts as an inhibitor of P-glycoprotein, and therefore it can be used also separately (without a conjugated drug) in combination with low molecular weight drugs to overcome multiple drug resistance of tumours to chemotherapy. Due to biodegradation of the block polymer system in the target tumour cells, PPO is released which is in itself a highly active inhibitor of P-glycoprotein. By degradation of the polymer system its ability to inhibit MDR is therefore significantly potentiated, whereas any transport of PPO other than within the conjugated amphiphilic polymer is not possible. PPO itself forms a micro-emulsion in aqueous solutions that is unusable for medical use *in vivo.* Moreover, if this polymer system comprises a conjugated drug, also the drug is released only after pH has changed, i.e. in the target tumour tissue. Therefore, the drug is transported by controlled delivery to the tumour tissue, where it is released and in addition, the potential resistance of the tumour tissue to this drug is inhibited by the block copolymer itself or PPO released therefrom. The whole system of the present invention thus allows for a significant reduction of MDR and controlled delivery of the drug to the tissue, thus reducing the effective doses of drug administered to the subject being treated.

The subject of the present invention is a block copolymer for overcoming drug resistance of tumours to chemotherapy, comprising at least one block A and at least one block B, wherein at least one block A and at least one block B are connected to each other with a linker L, which is hydrolytically, enzymatically and/or reductively biodegradable and selected from a group comprising oligopeptides with two to five amino acids and/or a disulfide bond, and
wherein the hydrophilic block A is formed by poly(N-(2-hydroxypropyl) methacrylamide), eventually containing up to 20 mol% of monomer units of general formula (I), 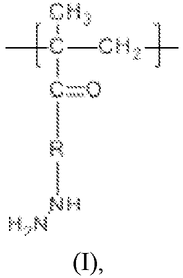
where R is aminoacyl selected from the group consisting of glycyl, glycylglycyl, β-alanyl, 6-aminohexanoyl, 4-aminobenzoyl, acyles derived from oligopeptides of two to five amino acids, especially GlyPheGly, GlyLeuGly, GlyLeuPheGly and GlyPheLeuGly;
and/or up to 15 mol% of monomer units of general formula (II), 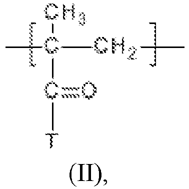
where T is oligopeptide attached to the monomer unit through an amide bond, wherein the oligopeptide is selected from the group consisting of the oligopeptides of two to five amino acids, preferably the oligopeptide is GlyPheGly, GlyLeuGly, GlyLeuPheGly, GlyPheLeuGly, AspProLys, AspProSer, and AspProGly; the block A may optionally contain terminal groups defined below;
and the hydrophobic block B is formed by a polypropylene oxide; the block B may optionally contain terminal groups as defined below;
wherein the block A has its molecular weight *Mₙ* in the range of from 4,000 to 60,000 g/mol (corresponding to 25 to 400 monomer units), preferably *Mₙ* is in the range of from 5,500 to 40,000 g/mol (37 to 260 monomer units), and the block B has its molecular weight *Mₙ* in the range of from 1,000 to 17,600 g/mol (23 to 400 monomer units), preferably a molecular weight from 1,500 to 12,000 g/mol (34 to 270 monomer units); and wherein the molecular weight of the block copolymer is in the range of from 5,000 to 95,000 g/mol.

The block copolymer for overcoming drug resistance of tumours to chemotherapy may further comprise terminal groups, wherein the terminal group of block A is 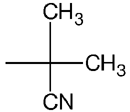 and the terminal group of block B is selected from the group consisting of -NH₂, dibenzocyclooctyle (-DBCO), 2-aminopropyl, and an oligopeptide of two to five amino acids connected to PPO through an amide bond, preferably the oligopeptide is GlyPheGly, GlyLeuGly, GlyLeuPheGly, GlyPheLeuGly, AspProLys, AspProSer, and AspProGly.

In one embodiment, blocks A and B are arranged in a linear or branched topology; preferably blocks A and B are arranged in the order of A-L-B or A-L-B-L-A or B-L-A-L-B, or according to general formula (III)

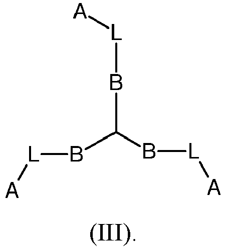

In another embodiment, the linker L is selected from the group comprising oligopeptides with two to five amino acids and/or a disulfide bond, preferably the linker L is oligopeptide AspProLys, AspProLysProAsp, GlyLeuGly, GlyPheGly, GlyPheLeuGly, GlyLeuPheGly or a disulfide bond. The biodegradable linker L may be attached to blocks A and B by an amide bond or as a part of the linking chain of general formula (IV) 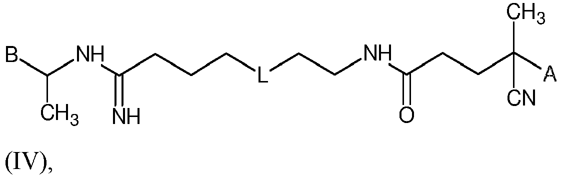 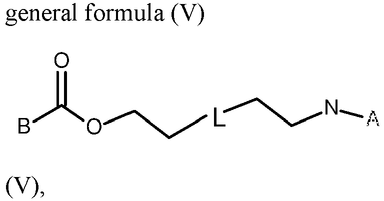 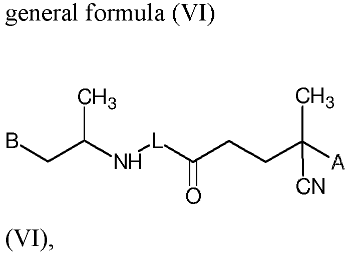 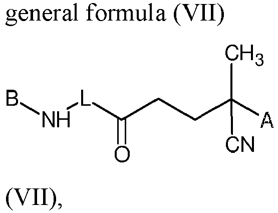 wherein L is oligopeptide of two to five amino acids and/or a disulfide bond, A represents the hydrophilic block A, and B represents the hydrophobic block B.

The subject of the present invention is also a polymer conjugate, which comprises the block copolymer according to the invention, and which further contains from 1 to 25 wt% of a low molecular weight drug covalently bound to the monomer unit of the general formula (I) and/or to the monomer unit of the general formula (II) of the block A by a hydrolytically and/or enzymatically degradable covalent bond, preferably by a hydrolytically degradable pH-sensitive bond, preferably the drug is a low molecular weight cytostatic, most preferably the low molecular weight cytostatic is selected from the group comprising doxorubicin, docetaxel, paclitaxel, pirarubicin, mitomycin C, daunorubicin, larotaxel. The low molecular weight drug means a drug that has a molecular weight ranging from 200 to 1,000 g/mol.

In one embodiment, the molecule of the low molecular weight drug is bound to the monomer unit of the general formula (I) and/or to the monomer unit of the general formula (II) of the block A of a polymer conjugate by a hydrazone or a peptide bond.

In one embodiment, the block copolymer according to the present invention and/or the polymer conjugate according to the present invention, are in an aqueous medium in the form of a micelle. The aqueous medium comprises water, saline, body fluids, particularly blood and blood plasma. The size and structure of the micelles depends on the hydrophilicity and hydrophobicity of blocks A and B and on their length. The hydrophilic coating layer of the micelle is formed by the block A, eventually with a bound drug, and the hydrophobic core is formed by the block B.

The subject of the present invention is also a method of preparation of the block copolymer according to the present invention, the said method comprising the following steps:
- a step of radical polymerization of N-(2-hydroxypropyl)methacrylamide, eventually containing up to 20 mol% of the monomer of the general formula (I), where R is aminoacyl selected from the group consisting of glycyl, glycylglycyl, β-alanyl,6-aminohexanoyl, 4-aminobenzoyl, acyles derived from oligopeptides of two to five amino acids, particularly GlyPheGly, GlyLeuGly, GlyLeuPheGly and GlyPheLeuGly, protected on hydrazide groups by tert-butyloxycarbonyl (Boc) groups, and/or optionally containing up to 15 mol% of the monomer of the general formula (II), where T is oligopeptide attached to the monomer unit by an amide bond, wherein the oligopeptide is selected from the group consisting of oligopeptides of two to five amino acids, preferably the oligopeptide is GlyPheGly, GlyLeuGly, GlyLeuPheGly, GlyPheLeuGly, AspProLys , AspProSer and AspProGly;
   at a temperature ranging from 40 to 100°C, preferably from 50 to 80°C, and in a solvent preferably selected from the group comprising of DMSO, aqueous buffers, dimethylacetamide, dimethylformamide, methanol, ethanol, dioxane, tetrahydrofuran, propanol, tert-butanol or mixtures thereof, initiated with an initiator, preferably selected from the group comprising ABIK-TT and ABIK-N₃, where ABIK-TT is 2-[(Z)-[1-cyano-1-methyl-4-oxo-4-(2-thioxothiazolidin-3-yl)butyl]azo]-2-methyl-5-oxo-5-(2-thioxothiazolidin-3-yl)pentanenitrile and ABIK-N₃ is *N-*(3-azidopropyl)-4- [(Z)- [4-(3-azidopropylamino)-1-cyano-1-methyl-4-oxo-butyl]azo]-4-cyano-pentanamide, optionally in the presence of a chain transfer agent, preferably selected from the group of CTA-TT and CTA-N₃, where CTA-TT is [1-cyano-1-methyl-4-oxo-4-(2-thioxothiazolidin-3-yl)butyl]benzencarbodithioate and CTA-N₃ is [4-(3-azidopropylamino)-1-cyano-1-methyl-4-oxo-butyl]benzencarbodithioate, to form the hydrophilic block A as a semitelechelic hydrophilic polymer block A, preferably with a terminal TT or N₃ group; wherein the terminal TT or N₃ group of the resulting semitelechelic hydrophilic polymer block A may further react with aminoethylpyridyldisulfide to give semitelechelic hydrophilic polymer block A with a terminal aminoethylpyridyldisulfide (PDS) group, or with a peptide of two to five amino acids to give a semitelechelic hydrophilic polymer block A with a terminal peptide chain, as a precursor of the linker L;
- a step in which the semitelechelic hydrophilic polymer block A from the previous step reacts with the semitelechelic or telechelic hydrophobic polymer block B of general formulas Y-PPO-Y, Y-L-PPO-L-Y or PPO-L-Y or 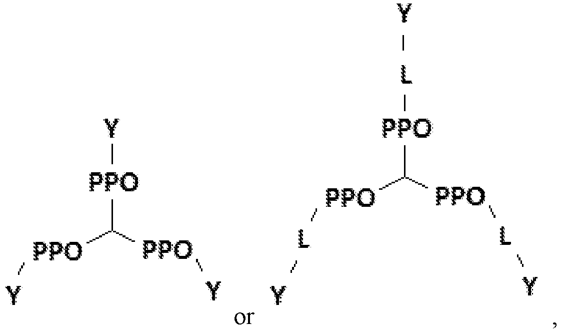 wherein Y is -NH₂, oligopeptide of two to five amino acids, succinimidyl carbonate or dibenzocyclooctyne group, and wherein L is oligopeptide of two to five amino acids or an -S-S- bond, and wherein L may be attached to the block B and group Y by an amide bond or as a part of the linking chain of general formula (VIII) 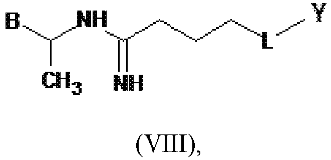 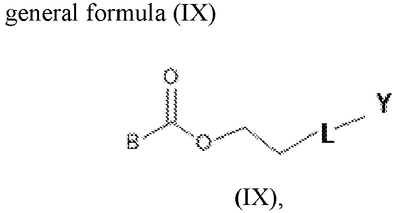 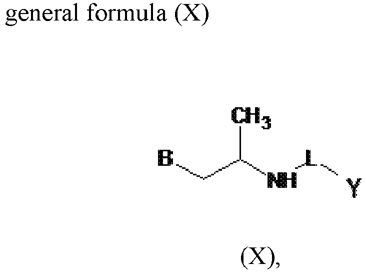 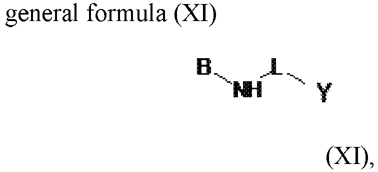 or with the chain transfer agent PPO-(L-CTA)₂, where L is peptide of two to five amino acids or -S-S- bond,
   to give an amphiphilic block copolymer according to the invention, which may be optionally further subjected to purification by column chromatography and/or removal of protecting Boc groups, by e.g. trifluoroacetic acid, and/or lyophilization. Lyophilization of the resulting product allows the amphiphilic copolymer to be stored without the risk of its decomposition, and allowing immediate formation of micelles after its dissolution. Types of reactions that are preferably used for preparing the amphiphilic block copolymer according to the present invention are polycondensation (e.g. using the commercial NH₂-PPO-NH₂ or PPO-(oligopeptide)₂), "click" reaction (e.g. using terminal groups of dibenzylcyclooctyne, propargyl and azide, respectively, at semitelechelic blocks A and B respectively), and RAFT polymerization (using the pre-prepared chain transfer agent, e.g. PPO-(CTA)₂). While NH₂-PPO-NH₂ is commercially available, other derivatives of the general formula Y-PPO-Y may be prepared by activation of the terminal OH groups of the commercially available PPO, which is carried out by reaction with phosgene, N-hydroxysuccinimide and *N,N-*diisopropylethylamine, thereby converting the terminal groups to succinimidyl carbonate groups which optionally may be further converted to dibenzocyclooctyne or propargyl groups by reaction with DBCO-amine or propargyl amine and NN-dii sopropyl ethyl amine, or which may be modified by trifluoroacetate of oligopeptide, preferably of two to five amino acids, and *N,N*-diisopropylethylamine.

The subject of the present invention is also a method for preparing a polymer conjugate according to the invention, which comprises the following steps:
- a step of radical polymerization of N-(2-hydroxypropyl)methacrylamide, optionally containing up to 20 mol% of the monomer of the general formula (I), where R is aminoacyl selected from the group consisting of glycyl, glycylglycyl, β-alanyl,6-aminohexanoyl, 4-aminobenzoyl, acyles derived from the oligopeptides of two to five amino acids, especially GlyPheGly, GlyLeuGly, GlyLeuPheGly and GlyPheLeuGly, protected on hydrazide groups by tert-butyloxycarbonyl (Boc) groups, and/or optionally containing up to 15 mol% of the monomer of the general formula (II), where T is oligopeptide attached to the monomer unit through an amide bond, wherein the oligopeptide is selected from the group consisting of the oligopeptides of two to five amino acids, preferably oligopeptide is GlyPheGly, GlyLeuGly, GlyLeuPheGly, GlyPheLeuGly, AspProLys , AspProSer and AspProGly; wherein the monomer of the general formula (I) and/or the monomer of the general formula (II) may eventually contain a covalently bound molecule of a low molecular weight drug, preferably selected from the group comprising doxorubicin, docetaxel, paclitaxel, pirarubicin, mitomycin C, daunorubicin, larotaxel; the eventual molecule of a low molecular weight drug is bound by a biodegradable (enzymatically or hydrolytically cleavable) bond;
   at a temperature ranging from 40 to 100°C, preferably from 50 to 80°C, and a solvent preferably selected from the group comprising of DMSO, aqueous buffers, dimethylacetamide, dimethylformamide, methanol, ethanol, dioxane, tetrahydrofuran, propanol, tert-butanol or mixtures thereof, initiated with an initiator, preferably selected from the group consisting of ABIK-TT and ABIK-N₃, where ABIK-TT is 2-[(Z)-[1-cyano-1-methyl-4-oxo-4-(2-thioxothiazolidin-3-yl)butyl]azo] -2-methyl-5-oxo-5-(2-thioxothiazolidin-3-yl)pentanenitrile and ABIK-N₃ is *N-*(3-azidopropyl)-4- [(Z)- [4-(3 -azidopropylamino)-1-cyano-1-methyl-4-oxo-butyl]azo]-4-cyano-pentanamide, optionally in the presence of a chain transfer agent, preferably selected from the group of CTA-TT and CTA-N₃, where CTA-TT is [1-cyano-1-methyl-4-oxo-4-(2-thioxothiazolidin-3-yl)butyl]benzencarbodithioate and CTA-N₃ is [4-(3-azidopropylamino)-1-cyano-1-methyl-4-oxo-butyl]benzencarbodithioate, to form the hydrophilic block A, preferably with a terminal TT or N₃ group; optionally containing a covalently bound low molecular weight drug, as a semitelechelic hydrophilic polymer block A, optionally containing a covalently bound low molecular weight drug; wherein the terminal TT or N₃ group of the resulting semitelechelic hydrophilic polymer block A may further react with aminoethylpyridyldisulphide to give a semitelechelic hydrophilic polymer block A with terminal aminoethylpyridyldisulphide (PDS) group, or with a peptide to form a semitelechelic hydrophilic polymer block A with a terminal peptide chain or PDS group as a precursor of the linker L;
- a step in which the semitelechelic hydrophilic polymer block A from the previous step, eventually containing the covalently bound low molecular weight drug, reacts with the semitelechelic or telechelic hydrophobic polymer block B of general formula Y-PPO-Y, Y-L-PPO-L-Y or PPO-L-Y or 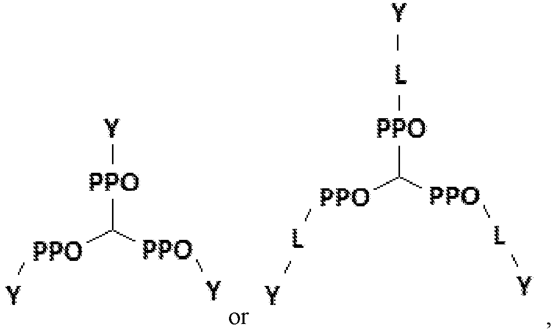 where Y is -NH₂, oligopeptide of two to five amino acids, succinimidyl carbonate or dibenzocyclooctyne group, and where L is peptide with two to five amino acids or -S-S- bond, wherein L may be attached to the block B and group Y by an amide bond or as a part of the linking chain of formulas (VIII), (IX), (X), (XI),
   or with the chain transfer agent PPO-(L-CTA)₂, where L is peptide of two to five amino acids or -S-S- bond,
   to give an amphiphilic block copolymer of the invention or, optionally, if the hydrophilic block A from the previous step already contains a covalently bound drug, to give a polymer conjugate of the invention. While NH₂-PPO-NH₂ is commercially available, other derivatives of formulas Y-PPO-Y, Y-L-PPO-L-Y, PPO-L-Y and trimers can be prepared by activation of the terminal OH groups of the commercially available PPO (HO-PPO-OH or poly(propylene oxide)triol PPO (OH)₃), which is carried out by reaction with phosgene, N-hydroxysuccinimide and *N,N-*diisopropylethylamine, thereby converting the terminal groups to succinimidyl carbonate groups which optionally may be further converted to dibenzocyclooctyne groups by reacting with DBCO-amine and *N,N-*diisopropylethylamine, or which may be modified by trifluoroacetate of oligopeptide, preferably of two to five amino acids, and *N,N-*diisopropylethylamine;
- optionally a step of removal of protecting Boc groups, when the resulting amphiphilic block copolymer or polymer conjugate from the previous step reacts e.g., with trifluoroacetic acid;
- optionally a step of conjugation of free hydrazide groups and/or amide groups of monomer units of the general formula (I) or (II) of the amphiphilic block copolymer, resulting from one of the previous two steps, with a low molecular weight drug in its free form or in a form of its salt with an acid, e.g. HCl, or of a derivative thereof, e.g. with levulinic acid; wherein the low molecular weight drug is a drug which has a molecular weight ranging from 200 to 1,000 g/mol, preferably the low molecular weight drug is doxorubicin, docetaxel, paclitaxel, pirarubicin, mitomycin C, daunorubicin, larotaxel;
- optionally a step of purification by column chromatography and/or lyophilization of the resulting product from the previous step.

The subject of the present invention is also a pharmaceutical composition comprising:
the block copolymer according to the invention and the low molecular weight drug in its free form (meaning a drug non-covalently bound to the block copolymer of the invention) that has a molecular weight ranging from 200 to 1,000 g/mol, preferably the drug is a low molecular weight cytostatic, most preferably a low molecular weight cytostatic selected from the group comprising doxorubicin, docetaxel, paclitaxel, pirarubicin, mitomycin C, daunorubicin, larotaxel, in its free form; and/or the polymer conjugate according to the invention;
wherein the pharmaceutical composition further comprises at least one pharmaceutically acceptable ingredient selected from the group comprising antiadhesives, binders, coating agents, colouring agents, disintegrants, flavourings, lubricants, preservatives, sweeteners, absorbents. Preferably, the block copolymer and/or the polymer conjugate in the pharmaceutical composition are present in a lyophilized form which enables its rapid reconstitution into micellar form after dissolution in an aqueous medium.

The subject of the present invention is also the block copolymer according to the invention and/or the polymer conjugate according to the invention and/or the pharmaceutical composition according to the invention for use as a medicament.

The subject of the present invention is also the block copolymer according to the invention and/or the polymer conjugate according to the invention and/or the pharmaceutical composition according to the invention for use as a cytostatic.

The subject of the present invention is also the block copolymer according to the invention and/or the polymer conjugate according to the invention and/or the pharmaceutical composition according to the invention for use as a medicament in the treatment of solid tumours and/or lymphoma and/or leukaemia.

The presence of a hydrolytically and/or enzymatically and/or reductively biodegradable linker L as defined in the claims between the blocks A and B highly significantly and unexpectedly increases the inhibiting activity of P-gp of the resulting amphiphilic block copolymer and hence its cytostatic effectiveness as compared with systems in which there is only a non-degradable covalent bond between the hydrophobic block B and hydrophilic block A. As is apparent from Examples 35 to 38 of the invention (see below), by replacing a covalent bond between blocks A and B with a biodegradable oligopeptide of two to five amino acids or -S-S- bond, the resulting inhibiting ability of the polymer towards P-gp on the multiresistant tumour lines increases twice, which significantly increases also the cytotoxicity of the degradable polymer conjugate itself against resistant tumour cells. The biodegradable amphiphilic block copolymer, when added to a free drug or other polymer pro-drug, will increase cytotoxicity significantly more than a non-degradable amphiphilic block copolymer. Due to degradation of the polymer system according to the invention in the target tumour cells, PPO itself is released, which is in itself a highly active inhibitor of P-glycoprotein, better than the amphiphilic block copolymer, in which the hydrophilic part prevents PPO from being fully effective. It must be emphasized that the delivery of PPO to the target tissues other than within a conjugated amphiphilic polymer is not possible, since PPO itself forms in aqueous solutions a micro-emulsion unusable for medical use *in vivo.* Within a degradable block copolymer, hence the conjugate, unique properties of the carrier system, namely the ability to targetedly accumulate in tumour tissue and deliver and release a drug in this tissue, combine with a unique ability to inhibit multiple drug resistance by highly active parts of the system after its degradation. The inhibition of MDR unexpectedly and significantly increases by degradation of the polymer system according the present invention in the target tissue (i.e. biodegradation of the linker L).

### Brief Description of Figures

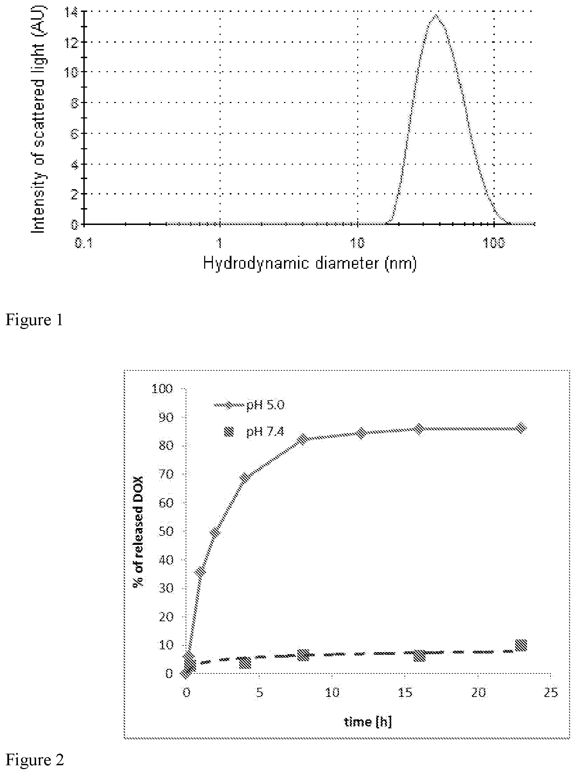

Figure 1: Example of a particle size distribution curve depending on the intensity of scattered light.
Figure 2: Release of the drug doxorubicin from the diblock polymer conjugate with doxorubicin as prepared in Example 28A at 37°C in buffers of pH 5.0 and 7.4.

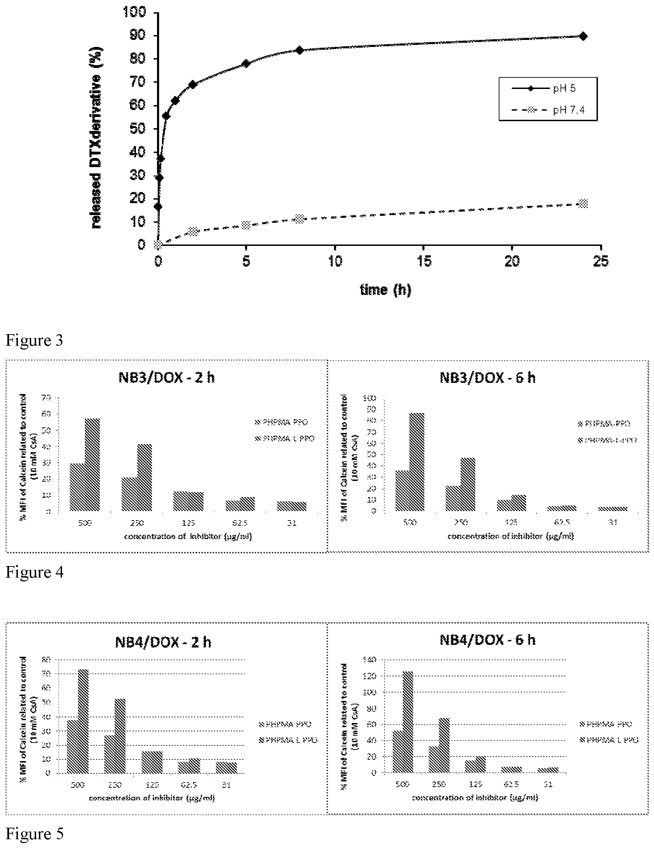

Figure 3: Dependence of the rate of release of docetaxel derivative (DTX-LEV) from the solution of micellar polymer conjugate with the bound DTX-LEV of Example 28B using a pH-labile hydrazone bond in phosphate buffers of pH 5.0 and pH 7.4 at 37 °C.
Figure 4: Calcein assay in NB3/DOX cell line: The degree of inhibition of P-gp mediated efflux by various concentrations of diblock degradable polymer PHPMA-L-PPO, where L is disulphide linker, with regard to non-degradable PHPMA-PPO (Example 20) after 2 and 6 hours incubation. Expressed as a percentage of calcein MFI related to the control, control 10 µM CsA (cyclosporine A).
Figure 5: Calcein assay in NB4/DOX cell line: The degree of inhibition of P-gp mediated efflux by various concentrations of diblock degradable polymer PHPMA-L-PPO, where L is disulphide linker, with regard to non-degradable PHPMA-PPO (Example 20) after 2 and 6 hours of incubation. Expressed as a percentage of calcein MFI related to the control, control 10 µM CsA (cyclosporine A).

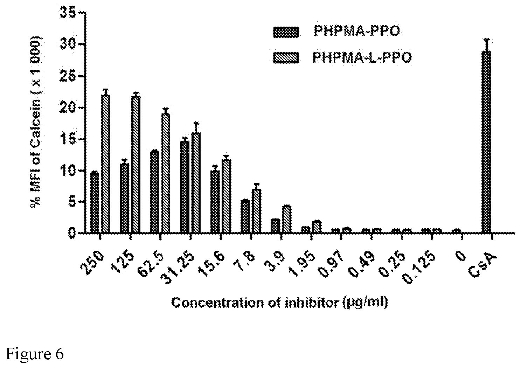

Figure 6: Calcein assay in P388/MDR cell line: The degree of inhibition of P-gp mediated efflux by various concentrations of diblock degradable polymer PHPMA-L-PPO, where L is AspProLys linker, with regard to non-degradable PHPMA-PPO (Example 20) after 6 h incubation. Expressed as a percentage of calcein MFI related to the control, control 10 µM CsA (cyclosporine A).

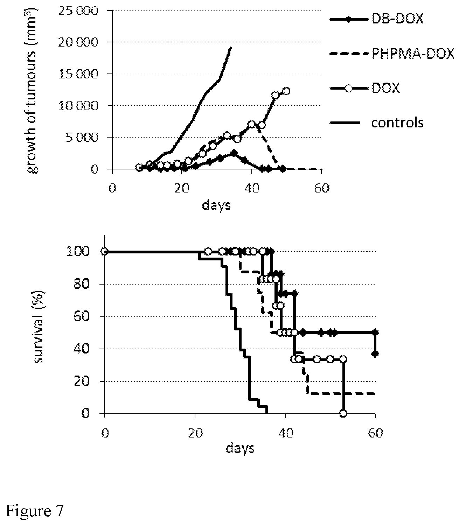

Figure 7: Average tumour growth (a) and survival (b) in C57BL/6 mice with EL4 lymphoma after treatment with micellar diblock conjugate of Example 28 (DB-DOX) or linear conjugate with doxorubicin (PHPMA-DOX) or free doxorubicin (as described in Table 7). The tumour size is recorded as volume V=a*b²/2, where "a" is the longer diameter of the tumour and "b" is the shorter one.

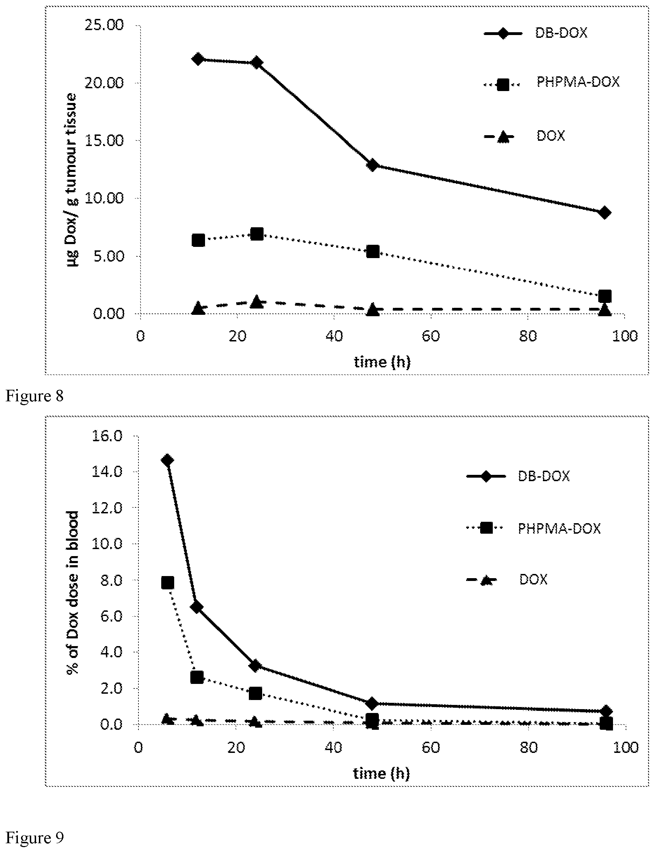

Figure 8: Time dependence of DOX amount accumulated in solid tumours of EL4 T cell lymphoma in mice after administration of the diblock polymer conjugate with DOX of Example 28 (DB-DOX). As a control group was used the linear PHPMA-based conjugate with DOX (PHPMA-DOX) and the free drug (DOX), dosage 10 mg Dox/kg, free drug 2 x 5 mg DOX/kg.
Figure 9: Time dependence of DOX amount circulating in the bloodstream of mice inoculated with EL4 T-cell lymphoma after administration of the diblock polymer conjugate with DOX of Example 28 (DB-DOX). As a control group was used the linear PHPMA based conjugate with DOX (PHPMA-DOX) and the free drug (DOX), dosage 10 mg Dox/kg, free drug 2 x 5 mg Dox/kg.

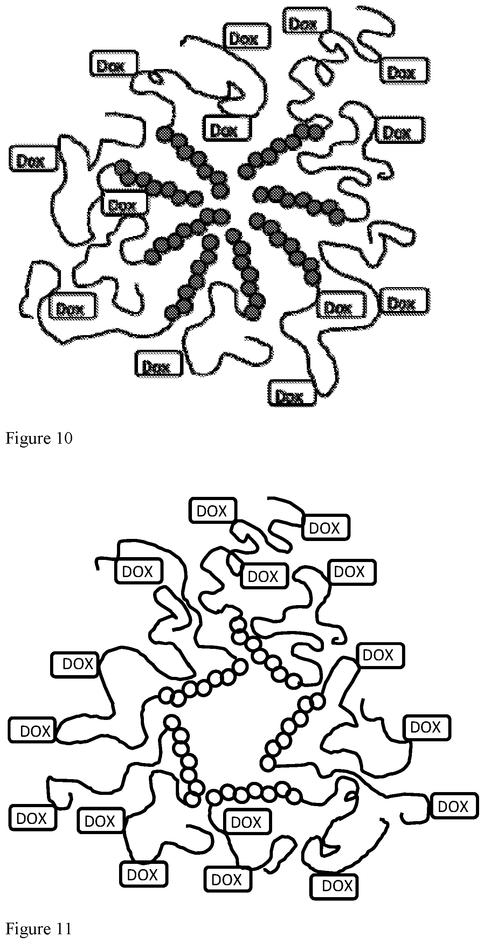

Figure 10: Scheme of a micelle formed by the A-L-B type amphiphilic diblock copolymer with a covalently bound drug doxorubicin (DOX).
Figure 11: Scheme of a micelle formed by the A-L-B-L-A type amphiphilic triblock copolymer with a covalently bound drug doxorubicin (DOX).

### Examples

### Example 1: Synthesis of the chain transfer agent PPO-(GFLG-CTA)₂ for RAFT polymerization

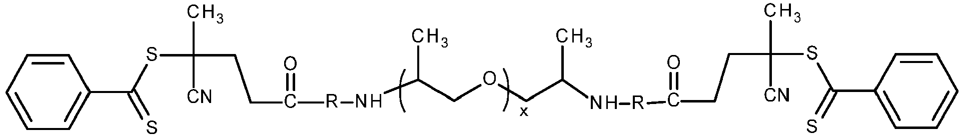

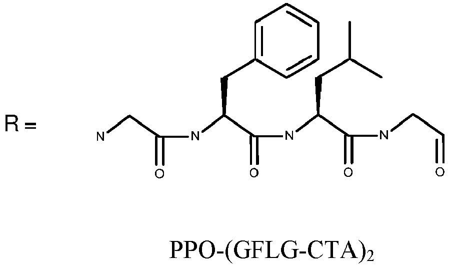

The commercially available poly(propylene oxide)-bis-2-aminopropylether was first modified at both terminal amino groups by tetrapeptide GFLG prepared by solid-phase peptide synthesis. NH₂-PPO-NH₂ (M ~ 4,000 g/mol; 8.60 mg, 4.30 x 10⁻³ mmol -NH₂) was dissolved in DMF and the solution was added to excess of GFLG (7.29 mg; 17 x 10⁻³ mmol) and solid N-hydroxysuccinimide (NHS, 1.96 mg; 17 x 10⁻³ mmol). The reaction mixture was cooled using an ice water bath. The excess of *N,N*-diisopropylcarbodiimide (DIC) was added to the reaction mixture and the reaction mixture was reacted under stirring overnight. The product PPO(GFLG)₂ was purified by column chromatography (LH-20, MeOH) and after solvent evaporation it was dried to constant weight. The content of tetrapeptide on the polymer was determined by amino acid analysis. Yield: 74%. The polymer chain transfer agent *PPO-(GFLG-CTA)₂* usable for controlled radical polymerization was then prepared by reaction of PPO(GFLG)₂ with 2.5 fold excess of commercially available succinimide ester of 4-cyano-4-(phenylcarbonothioylthio)pentanoic acid (CTA-NHS; 22.8 mg, 60.5 x 10⁻³ mmol). NH₂-PPO-NH₂ was dissolved in 0.5 mL of dichloromethane (DCM) and this solution was added to a suspension of CTA-NHS in 0.2 mL of DCM. The reaction mixture was allowed to react with stirring overnight.

The crude product was purified on silica gel using a mixture solvent of chloroform/ethyl acetate (5/1) and chloroform/methanol (5/1). The solvent was evaporated and the product was dried under vacuum to constant weight. The product was characterized by TLC, HPLC and SEC. The functionality of terminal DTB groups of the final product was 1.96. Unless otherwise stated in what follows, all of the starting monomers and following polymers are always racemic mixtures.

### Example 2: Synthesis of semitelechelic random copolymer poly(HPMA-co-MA-AH-NHNH-Boc)-TT (hydrophilic block A) using solution radical copolymerization

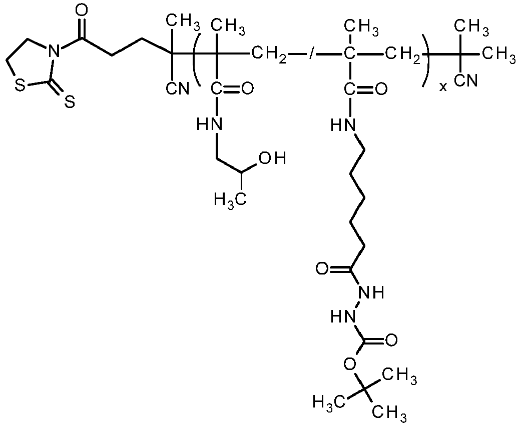

### poly(HPMA-co-MA-AH-NHNH-Boc)-TT

Semitelechelic random copolymer poly(HPMA-co-MA-AH-NHNH-Boc)-TT, containing terminal thiazolidine-2-thione (TT) groups and protected hydrazide groups (-NHNH-Boc) along the polymer chain, was prepared by solution radical copolymerization of *N-(2-*hydroxypropyl) methacrylamide (HPMA) and methacryloyl-6-aminohexanoylhydrazide protected on hydrazide groups by tert-butyloxycarbonyl groups (MA-AH-NHNH-Boc) in DMSO at 60°C and initiated with initiator ABIK-TT (2-[(Z)-[1-cyano-1-methyl-4-oxo-4-(2-thioxothiazolidin-3-yl)butyl] azo]-2-methyl-5-oxo-5-(2-thioxothiazolidin-3-yl) pentanenitrile) (CZ 298 945). Characterization of the resulting copolymer: *Mₙ* (SEC) 12,400 g/mol; D ~ 1.60; *F*_{(TT)} 1.18; content (-NHNH₂) ~ 5.4 mol%; Rₕ ~ 4.4 nm. Following the procedure of Example 2, other copolymers poly(HPMA-co-MA-AH-NHNH-Boc)-TT were prepared analogously with different molecular weights *Mₙ* in the range from 4,000 to 58,000 g/mol, wherein the polydispersity index D was in all cases in the range from 1.5 to 1.8. The functionality of terminal TT groups was in the range from 110 to 120% (F_{(TT)} = 1.10 to 1.20). The content of -NHNH₂ groups along the chain (0.5 to 19.5 mol%). See Table 1.

**Table 1: Physico-chemical characterization of semitelechelic random copolymers poly(HPMA-co-MA-AH-NHNH-Boc)-TT prepared according to Example 2**

| Sample | *Mₙ* (g/mol) | Ð | *F*_{(TT)} | NHNH₂ groups (mol%) | *Rₕ* (nm) |
|---|---|---|---|---|---|
| 2-1 | 12,400 | 1.60 | 1.18 | 5.4 | 4.4 |
| 2-2 | 19,800 | 1.65 | 0.96 | 0.5 | 4.4 |
| 2-3 | 4,200 | 1.72 | 0.95 | 15.6 | 3.9 |
| 2-4 | 34,500 | 1.79 | 0.94 | 10.6 | 4.5 |
| 2-5 | 57,900 | 1.66 | 0.96 | 19.5 | 4.9 |

### Example 3: Synthesis of semitelechelic random copolymer poly(HPMA-co-MA--GFLG-DOX)-TT (hydrophilic block A) using solution radical copolymerization

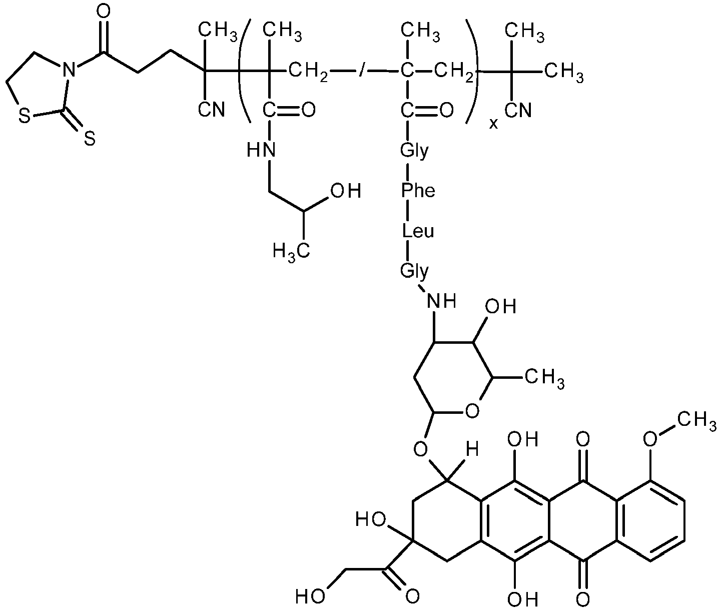

### poly(HPMA-co-MA-GFLG-DOX)-TT

Semitelechelic random copolymer poly(HPMA-co-MA-GFLG-DOX)-TT was prepared by solution radical copolymerization of HPMA and doxorubicin-containing monomer (N-methacryloylglycyl-DL-phenylalanylleucylglycyl)doxorubicin (Ma-GFLG-DOX), which was prepared by reaction of an equivalent amount of N-methacryloylglycyl-DL-phenylalanyl-L-leucylglycine 4-nitrophenyl ester (Ma-GFLG-ONp) with DOXHCl in DMF at 4 °C, in DMSO at 60°C and initiated with initiator ABIK-TT (2-[(Z)-[1-cyano-1-methyl-4-oxo-4-(2-thioxothiazolidin-3-yl)butyl]azo]-2-methyl-5-oxo-5-(2-thioxothiazolidin-3-yl)pentanenitrile). 220 mg (1.54 mmol) HPMA, 25 mg (0.025 mmol) MA-GFLG-DOX was dissolved in 1.7 mL DMSO and this solution was placed in a polymerization vial, which already has 84 mg (0.170 mmol) ABIK-TT (4 wt%) inside. The solution in the vial was bubbled with nitrogen for 15 min; the vial was sealed and placed in a thermostat heated to 60°C. The initiator dissolved after 3 min at 60°C. After 6 hours the reaction mixture was precipitated into 50 mL mixture of acetone - diethyl ether (2:1). The copolymer was dissolved in 3 mL of methanol and precipitated again into 50 mL of the same precipitation mixture. The precipitated copolymer was filtered through S4 sintered glass filter and dried until constant weight. Characterization of the copolymer poly(HPMA-co-MA-GFLG-DOX)-TT: *Mₙ* (SEC) 13,200 g/mol; *Ð* ~ 1.61; *F*_{(TT)} 1.20; content of GFLG monomer units: 3 mol%; content (DOX) ~ 8.9 wt%; *Rₕ* ~ 4.4 nm. Following the procedure of Example 3, other copolymers poly(HPMA-co-MA-GFLG-DOX)-TT were prepared analogously with different molecular weights *Mₙ* in the range from 4,500 to 59,500 g/mol, wherein the polydispersity index D was in all cases in the range from 1.5 to 1.8. The functionality of terminal TT groups was in the range from 110 to 120 % (F_{(TT)} = 1.10-1.20). The content of GFLG groups along the chain (0.5 to 15 mol%). See Table 2.

**Table 2: Physico-chemical characterization of semitelechelic random copolymers poly(HPMA-co-MA-GFLG-DOX)-TT prepared according to Example 5**

| Sample | *Mₙ* (g/mol) | *Ð* | F_{(TT)} | GFLG monomer unit (mol%) | *Rₕ* (nm) |
|---|---|---|---|---|---|
| 3-1 | 13,200 | 1.61 | 1.20 | 3.0 | 4.4 |
| 3-2 | 4,500 | 1.63 | 0.96 | 5.6 | 3.8 |
| 3-3 | 16,800 | 1.76 | 0.95 | 15.0 | 4.6 |
| 3-4 | 39,800 | 1.74 | 0.94 | 9.8 | 4.8 |
| 3-5 | 59,500 | 1.61 | 0.96 | 0.5 | 5.2 |

### Example 4: Synthesis of semitelechelic random copolymer raft-poly(HPMA-co-MA-AH-NHNH-Boc)-TT (hydrophilic block A) using controlled radical RAFT polymerization

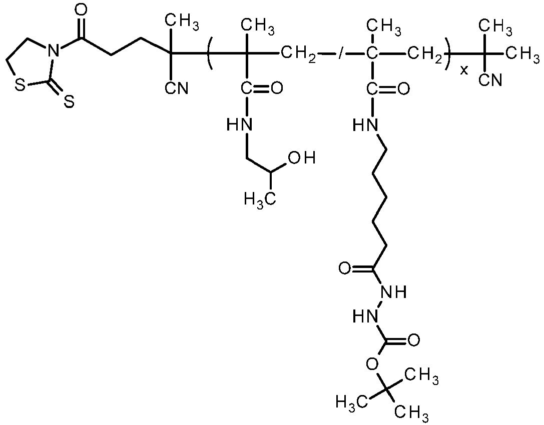

### raft-poly(HPMA-co-MA-AH-NHNH-Boc)-TT

Semitelechelic random copolymer raft-poly(HPMA-co-MA-AH-NHNH-Boc)-TT, containing terminal thiazolidine-2-thione (TT) groups and protected hydrazide groups (-NHNH-Boc) along the polymer chain, was prepared by controlled radical RAFT polymerization with the molar ratio of initiator/chain transfer agent/comonomers was 1/2/150. The ratio of comonomers HPMA/MA-AH-NHNH-Boc was 92/8 mol%. HPMA was dissolved in tert-butanol (6,747 mL), MA-AH-NHNH-Boc, initiator 2-[(Z)-[1-cyano-1-methyl-4-oxo-4-(2-thioxothiazolidin-3-yl) butyl]azo]-2-methyl-5-oxo-5-(2-thioxothiazolidin-3-yl)pentanenitrile (ABIK-TT) and chain transfer agent [1-cyano-1-methyl-4-oxo-4-(2-thioxothiazolidin-3-yl)butyl]benzencarbodithioate (CTA-TT) were dissolved in dimethyl sulphoxide (DMSO; 1,687 mL). These solutions were mixed together in a polymerization vial. The polymerization mixture was bubbled with argon for 10 min. Copolymerization was carried out at 70°C for 16 hours in a sealed vial. The product was isolated by precipitation into acetone/diethyl ether 1: 1, filtered off and dried to constant weight. Reactive ω-terminal dithiobenzoate (DTB) groups were removed by 2,2'-azobisisobutyronitrile (AIBN). Characterization of the copolymer raft-poly(HPMA-co-MA-AH-NHNH-Boc)-TT: *Mₙ* (SEC) 8,400 g/mol; *Ð* ~ 1.20; *F*_{(TT)} 0.98; content (-NHNH₂) ~ 5.9 mol%; conversion ~ 46% (833 mg); *Rₕ* ~ 4.5 nm.

Following the procedure of Example 4, other copolymers raft-poly(HPMA-co-MA-AH-NHNH-Boc)-TT were prepared analogously with different molecular weights *Mₙ* in the range from 4,000 to 60,000 g/mol, wherein the polydispersity index D was in all cases in the range from 1.1 to 1.2. The functionality of terminal TT groups was in the range from 90 to 99 % (F_{(TT)} = 0.90-0.99). The content of -NHNH₂ groups along the chain (0.5 to 20.5 mol%). See Table 3.

**Table 3: Physico-chemical characterization of semitelechelic random copolymers raft-poly(HPMA-co-MA-AH-NHNH-Boc)-TT prepared according to Example 4**

| Sample | *Mₙ* (g/mol) | *Ð* | *F*_{(TT)} | NHNH₂ groups (mol%) | *Rₕ* (nm) |
|---|---|---|---|---|---|
| 4-1 | 8,400 | 1.20 | 0.98 | 5.9 | 4.5 |
| 4-2 | 16,200 | 1.15 | 0.96 | 12.3 | 4.9 |
| 4-3 | 38,000 | 1.12 | 0.95 | 0.6 | 5.3 |
| 4-4 | 58,900 | 1.19 | 0.94 | 6.8 | 5.6 |
| 4-5 | 4,100 | 1.16 | 0.96 | 19.8 | 3.8 |

### Example 5: Synthesis of telechelic random copolymer raft-TT-poly(HPMA-co-MA-AH-NHNH-Boc)-TT (hydrophilic block A) using controlled radical RAFT polymerization

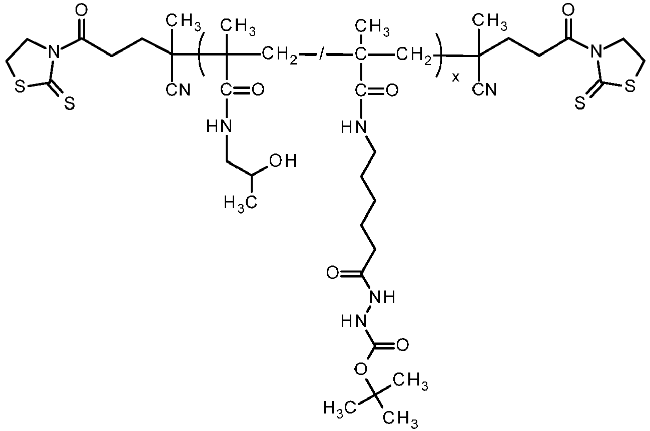

### raft-TT-poly(HPMA-co-MA-AH-NHNH-Boc)-TT

Telechelic random copolymer raft-TT-poly(HPMA-co-MA-AH-NHNH-Boc)-TT, containing terminal thiazolidine-2-thione (TT) groups and protected hydrazide groups (-NHNH-Boc) along the polymer chain, was prepared using controlled radical RAFT polymerization similarly as in Example 4.

Only the ω-terminal dithiobenzoate (DTB) groups were not removed using AIBN, but converted to TT as follows. 15% solution of polymer with ABIK-TT (10 wt%) in DMSO was prepared. The solution in the vial was bubbled with argon for 10 minutes, sealed and left for 3 hours at 80°C. The product was isolated by precipitation into the mixture of acetone/diethyl ether 1:1, filtered off and dried to constant weight. Characterization: *Mₙ* (SEC) 8,500 g/mol; *Ð* = 1.19; *F*_{(TT)} 1.7; content (-NHNH₂) = 5.9 mol%; *R*ₕ = 4.8 nm. Following the procedure of Example 5, other copolymers raft-TT-poly(HPMA-co-MA-AH-NHNH-Boc)-TT were prepared analogously with different molecular weights *Mₙ* in the range from 5,000 to 59,000 g/mol. See Table 4.

**Table 4: Physico-chemical characterization of telechelic random copolymers raft-TT-poly(HPMA-co-MA-AH-NHNH-Boc)-TT prepared according to Example 5**

| Sample | *Mₙ* (g/mol) | *Ð* | *F*_{(TT)} | NHNH₂ groups (mol%) | *Rₕ* (nm) |
|---|---|---|---|---|---|
| 5-1 | 8,500 | 1.19 | 1.70 | 5.9 | 4.8 |
| 5-2 | 5,000 | 1.18 | 1.75 | 15.6 | 4.2 |
| 5-3 | 18,200 | 1.16 | 1.76 | 0.9 | 5.0 |
| 5-4 | 35,600 | 1.15 | 1.69 | 18.6 | 5.3 |
| 5-5 | 58,600 | 1.16 | 1.67 | 9.8 | 5.6 |

### Example 6: Preparation of semitelechelic random copolymer raft-poly(HPMA-co-MA-AH-NHNH-Boc)-N₃ (hydrophilic block A) using RAFT polymerization

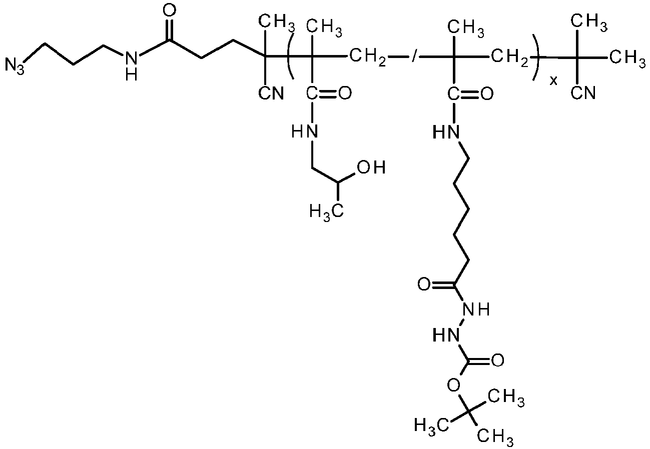

### raft-poly(HPMA-co-MA-AH-NHNH-Boc)-N₃

Semitelechelic random copolymer raft-poly(HPMA-co-MA-AH-NHNH-Boc)-N₃ with terminal azide groups was prepared in a similar manner as the semitelechelic PHPMA-based copolymer raft-poly(HPMA-co-MA-AH-NHNH-Boc)-TT in Example 4. The molar ratio of initiator/chain transfer agent/comonomers was 1/2/180. The ratio of comonomers HPMA/MA-AH-NHNH-Boc was 92/8 mol%. HPMA was dissolved in tert-butanol (6,747 mL), MA-AH-NHNH-Boc, initiator N-(3-azidopropyl)-4-[(Z)-[4-(3-azidopropylamino)-1-cyano-1-methyl-4-oxo-butyl]azo]-4-cyano-pentanamide (ABIK-N₃) and chain transfer agent [4-(3 -azidopropylamino)-1-cyano-1-methyl-4-oxo-butyl]benzencarbodithioate (CTA-N₃) were dissolved in dimethyl sulphoxide (DMSO; 1,687 mL). These solutions were mixed together in a polymerization vial. The polymerization mixture was bubbled with argon for 10 min. Copolymerization was carried out at 70°C for 16 hours in a sealed vial. The product was isolated by precipitation into the mixture of acetone/diethyl ether (1:1), filtered off and dried to constant weight. Reactive ω-terminal dithiobenzoate (DTB) groups were removed by 2,2'-azobisisobutyronitrile (AIBN). Characterization of the product: *Mₙ* (SEC) 9,100 g/mol; D ~ 1.21; F_{(N3)} 0.99; content (-NHNH₂) ~ 5.4 mol%; conversion ~ 63% (750 mg); *R*ₕ ~ 4.5 nm.

### Example 7: Synthesis of semitelechelic random copolymer raft-poly(HPMA-co-MA-AH-NHNH-Boc) PDS (hydrophilic block A)

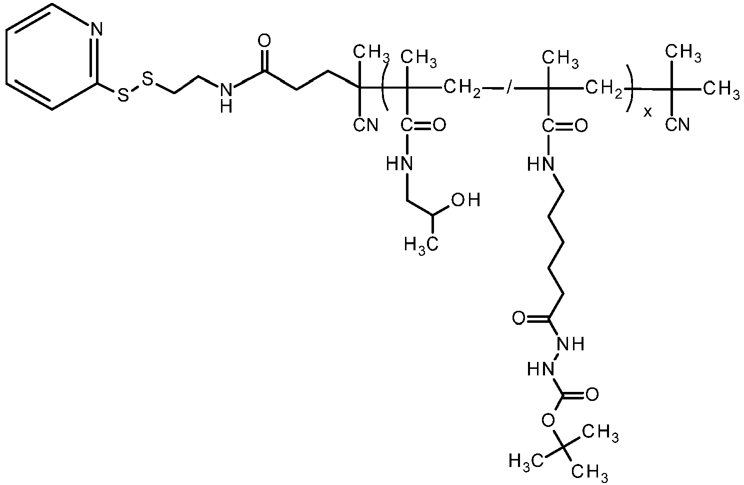

Semitelechelic random copolymer raft-poly(HPMA-co-MA-AH-NHNH-Boc)-PDS was prepared by reaction of semitelechelic random copolymer raft-poly(HPMA-co-MA-AH-NHNH-Boc)-TT (46.2 mg; 4.32 x 10⁻³ mmol TT groups), prepared according to Example 4, with an excess of aminoethylpyridyldisulphide (1.90 mg, 8.64 x 10⁻³ mmol) in DMA (0.5 mL) in the presence of a base (DIEA; 1.5 µL, 8.64 x 10⁻³ mmol). The crude product raft-poly(HPMA-co-MA-AH-NHNH-Boc)-PDS was purified by column chromatography (Sephadex LH-20, MeOH). The product was precipitated into diethyl ether and dried under vacuum to constant weight. The content of terminal PDS groups was determined by UV-VIS spectroscopy. Characterization: *Mₙ* (SEC) = 10,890 g/mol; D = 1.11; *F_{(PDS)}* = 0.80; content (-NHNH₂) = 5.8 mol%; conversion ~ 74% (34.7 mg); *R*ₕ = 4.7 nm.

### Example 8: Synthesis of semitelechelic random copolymer raft-poly(HPMA-co-MA-AH-NHNH-Boc)-GFLG (hydrophilic block A)

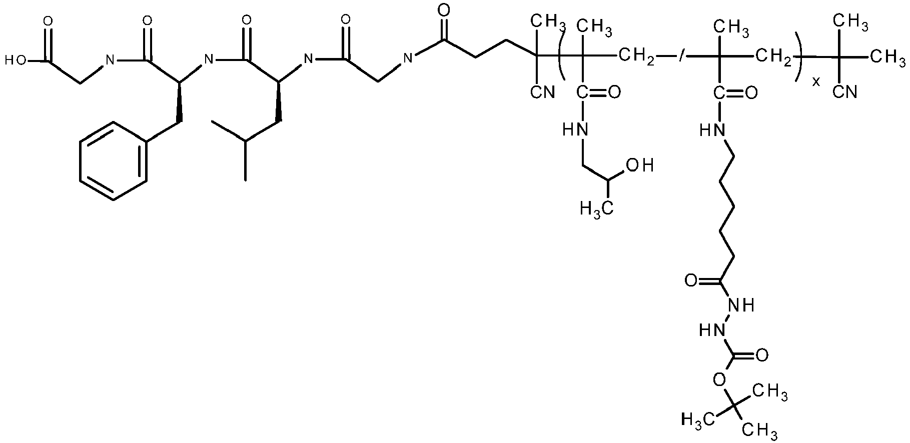

Tetrapeptide GlyPheLeuGly (GFLG-) was, like tripeptide Asp-Pro-Lys, prepared by solid-phase peptide synthesis. Semitelechelic copolymer raft-poly(HPMA-co-MA-AH-NHNH-Boc)-GFLG was prepared by modifying semitelechelic copolymer raft-poly(HPMA-coMA-AH-NHNH-Boc)-TT (Example 4) with tetrapeptide GFLG. Semitelechelic copolymer raft-poly(HPMA-co-MA-AH-NHNH-Boc)-TT (52.30 mg, 4.89 x 10⁻³ mmol -TT) was dissolved in DMA and added to an excess of of tetrapeptide GFLG (10.30 mg, 24 x 10⁻³ mmol). The reaction mixture was immediately treated with excess DIEA (16.74 µL, 9.78 x 10⁻³ mmol). The reaction mixture was allowed to react at room temperature overnight. The product was precipitated into diethyl ether, centrifuged, purified with LH-20 column in MeOH and dried under vacuum to constant weight. The sample was characterized by HPLC and SEC *(Mₙ* (SEC) = 10,770 g/mol; D = 1.10); content of tetrapeptide bound to the terminal TT group of the polymer was determined by amino acid analysis (*Mₙ* (calculated) = 10,933 g/mol; 3.59 wt%, i.e. 99% modification). Yield: 36.7 mg (68 %).

### Example 9: Synthesis of telechelic random copolymer raft-GFLGpoly(HPMA-co-MA-AH-NHNH Boc)-GFLG (hydrophilic block A) using controlled radical RAFT polymerization

Telechelic random copolymer raft-GFLG-poly(HPMA-co-MA-AH-NHNH-Boc)-GFLG was prepared analogously to semitelechelic copolymer of Example 8. Characterization: *Mₙ* (SEC) = 11,500 g/mol; D = 1.12; conversion (65 %).

### Example 10: Preparation of the poly(propylene oxide)-tris(succinimidyl carbonate) (PPO-TSC; hydrophobic block B)

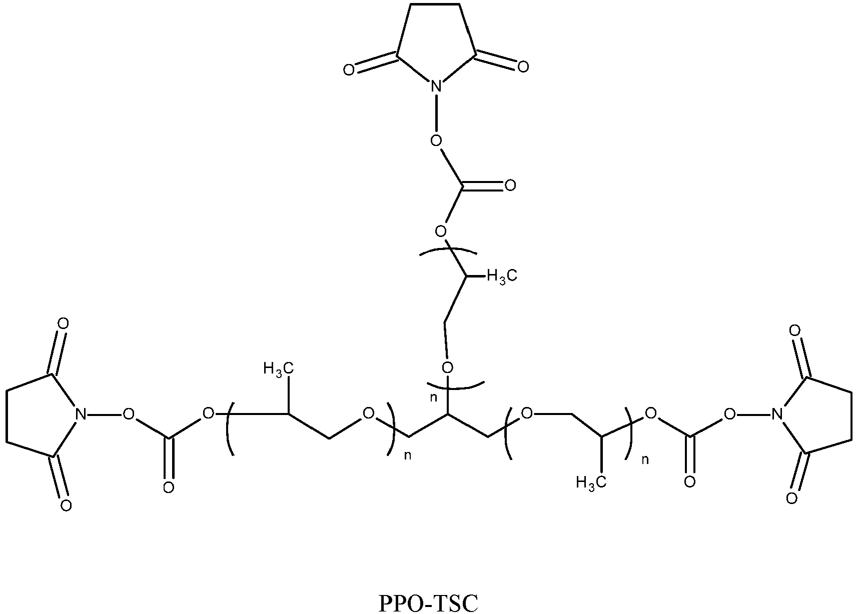

Poly(propylene oxide)-tris(succinimidyl carbonate) (PPO-TSC) was prepared according to the following procedure: the commercially available poly(propylene oxide)triol PPO(OH)₃ (Aldrich; M ~ 5,000 g/mol; 85 mg; 51 x 10⁻³ mmol -OH) was first twice dried by azeotropic distillation from dried, distilled toluene, then it was dissolved in toluene/DCM (0.637 mL/0.213 mL) and 20% solution of phosgene in toluene (0.5 mL) was added to the solution of PPO. The reaction mixture was allowed to react overnight. The following morning phosgene was evaporated to dryness under vacuum. The residue was dissolved into mixture of toluene/DCM (0.240 mL/0.120 mL), solid N-hydroxysuccinimide (58 mg, 0.504 mmol) and *N,N-*diisopropylethylamine (DIEA; 0.02 mL; 0.117 x 10⁻³ mmol) were added to the mixture and the mixture was allowed to react at room temperature on a shaker overnight. The solvent was evaporated and the product was dried under vacuum to constant weight. The product was used without any further purification in the next reaction (see Example 12).

### Example 11: Preparation of poly(propylene oxide)-bis(succinimidyl carbonate) (PPO-BSC; hydrophobic block B)

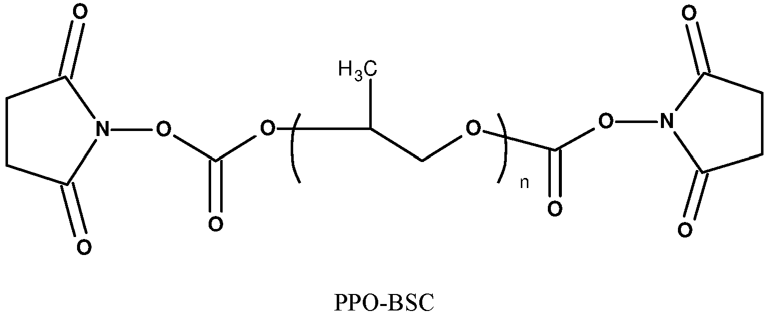

In the synthesis of poly(propylene oxide)-bis(succinimidyl carbonate) (PPO-BSC), the starting polymer is the commercially available poly(propylene oxide) (HO-PPO-OH) with M ~ 4,000 g/ mol. The preparation was analogous to the synthesis of PPO-TSC (see Example 10). The procedure in Example 11 was analogously used for activating terminal OH groups of polypropylene oxide) with molecular weights *Mₙ* 2,000 to 17,000 g/mol.

### Example 12: Preparation of PPO-(DBCO)3 (hydrophobic block B)

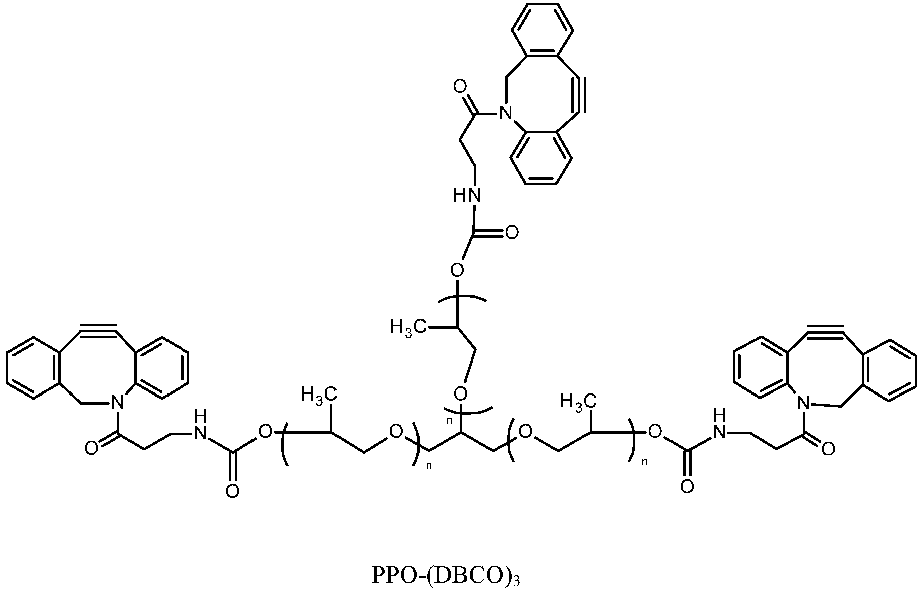

In the preparation of poly(propylene oxide) with terminal dibenzocyclooctyne groups (PPO (DBCO)₃), the starting polymer was PPO-TSC prepared in Example 10 (85 mg; 51 × 10⁻³ mmol TSC), which was dissolved in DCM. To a solution of polymer was added a solution of DBCO-amine (21.14 mg; 76.5 x 10⁻³ mmol NH₂) in DCM together with DIEA (0.026 mL; 153 x 10⁻³ mmol). The reaction mixture was allowed to react with stirring over the weekend. The solvent was evaporated under vacuum and the resulting product PPO-(DBCO)₃ was purified by column chromatography (Sephadex LH-20, methanol). The fraction with the sample was evaporated under vacuum and the product was dried to constant weight. Yield: 81% (80 mg). The content of DBCO groups was 71% (2.12 groups per polymer chain). Following the procedure of Example 12, PPO-BSC prepared in Example 11 was analogously modified to give the resulting PPO-(DBCO)₂.

### Example 13: Preparation of poly(propylene oxide) modified by tripeptide Asp-Pro-Lys (PPO-(Asp-Pro-Lys)₂; hydrophobic block B)

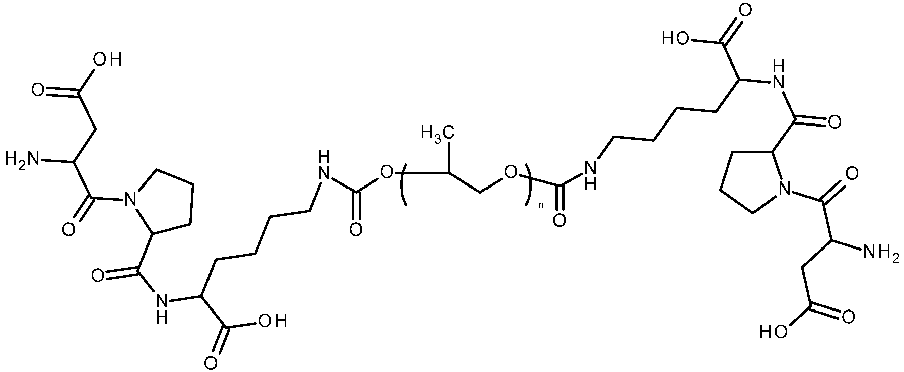

### PPO-(Asp-Pro-Lys)₂

PPO-(Asp-Pro-Lys)₂ was prepared by modifying PPO-BSC (Example 11) with tripeptide Asp-Pro-Lys. Trifluoroacetate of tripeptide Asp-Pro-Lys was prepared by solid-phase peptide synthesis. PPO-BSC (50 mg; 0.05 mmol SC groups) was dissolved in dimethylformamide (DMF) and added to excess of tripeptide Asp-Pro-Lys .TFA (34 mg; 0.1 mmol). The reaction mixture was immediately treated with excess of DIEA (51 µL, 0.3 mmol). The reaction mixture was allowed to react at room temperature overnight. The product was triturated with diethyl ether and further purified with LH-20 column in MeOH. The polymer fraction was evaporated to dryness under vacuum and the product was dried to constant weight. Yield: 47 mg (70%). Similarly, other biodegradable linkers were attached to PPO-BSC or PPO-TSC, e.g. Asp-Pro-Leu, Asp-Pro-Ser, GlyPheGly, GlyLeuGly, GlyLeuPheGly and GlyPheLeuGly.

### Example 14: Preparation of PPO-S-S DBCO (hydrophobic block B)

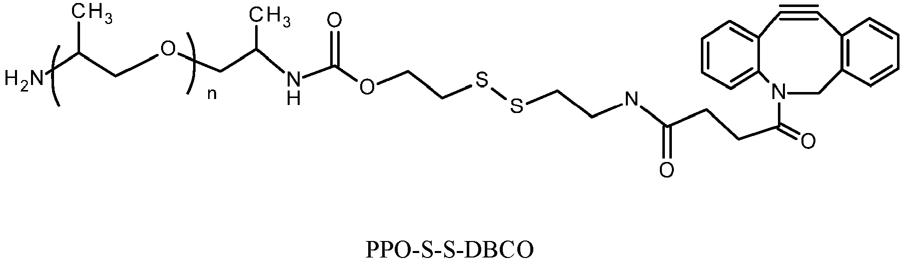

In the preparation of poly(propylene oxide) with terminal dibenzocyclooctyne groups (PPO-S-S-DBCO), the starting polymer was NH₂-PPO-NH₂ (30 mg; 7.5 x 10⁻³ mmol), which was dissolved in DMF. To a solution of NH₂-PPO-NH₂ (30 mg in 300 uL) was added a solution of DBCO-SS-NHS (4,2 mg; 7.4 x 10⁻⁶ mol) in DMF together with DIPEA (1.2 uL; 7 x 10⁻⁶ mol). The reaction mixture was allowed to react with stirring for 3 hours. The solvent was evaporated under vacuum and the resulting product PPO-S-S-DBCO was purified by column chromatography (Sephadex LH-20, methanol). The fraction with the sample was evaporated under vacuum and the product was dried to constant weight. Yield: 79% (26 mg).

### Example 15: Preparation of the A-L-B type amphiphilic diblock copolymer ([raft-poly(HPMA-co-MA-AH-NHNH-Boc)]-S-S-PPO-NH₂) with reductively and enzymatically cleavable S-S bond between the hydrophilic and hydrophobic blocks

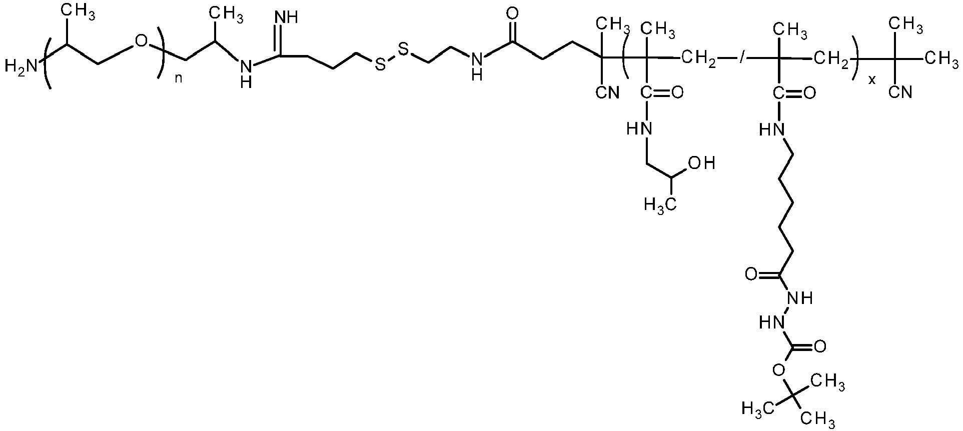

Poly(propylene oxide)-bis-2-aminopropylether (NH₂-PPO-NH₂; M ~ 4,000 g/mol; 11.33 mg; 5.66 x 10⁻³ mmol -NH₂) was dissolved in MeOH (0.1 mL) pre-bubbled with argon. This solution was added to the commercially available 2-iminothiolane (0.39 mg, 2.83 x 10⁻³ mmol -NH₂) and the reaction mixture was allowed to react. After 20 min the solution of raft-poly(HPMA-co-MA-AH-NHNH-Boc)-PDS (30 mg, 2.83 x 10⁻³ mmol) in MeOH (0.2 mL) was added to the reaction mixture and the reaction mixture was allowed to react for 2.5 hours. The product formation was monitored by SEC. The resulting diblock copolymer was purified by column chromatography (Sephadex LH-20, MeOH) and the solvent was evaporated under vacuum. The resulting product was dried to constant weight. Characterization of the prepared Boc-protected product: *Mₙ* (SEC) = 15,280 g/mol; D = 1.10; conversion ~ 65% (27 mg); *R*ₕ = 17.7 nm (in PBS).

### Example 16 Preparation of the A-L-B type diblock copolymer (raft-poly(HPMA-co-MA-AH-NHNH-Boc)-S-S-PPO-NH₂ II with reductively and enzymatically cleavable S-S bond between the hydrophilic and hydrophobic blocks, prepared by click reaction

The diblock copolymer (raft-poly(HPMA-co-MA-AH-NHNH-Boc)-S-S-PPO-NH₂ II was prepared by click reaction, without copper catalysis, of copolymer (raft-poly(HPMA-coMA-AH-NHNH-Boc)-N₃) (Example 6) and PPO-S-S-DBCO (Example 14). The copolymer raft-poly(HPMA-co-MA-AH-NHNH-Boc)-N₃ was dissolved in 160 µL of DMF. To this solution was added a solution of PPO-SS-DBCO (6.4 mg in 64 uL of DMF) and the reaction mixture was allowed to react overnight under constant stirring. The solvent was evaporated under vacuum and the resulting product was purified by column chromatography (Sephadex LH-20, methanol). The fraction with the sample was evaporated under vacuum and the product was dried to constant weight.

### Example 17: Preparation of the A-L-B type amphiphilic diblock copolymer ([raft-poly(HPMA-co-MA-AH-NHNH-Boc)]-GFLG-PPO-NH₂) with enzymatically cleavable bond between the hydrophilic and hydrophobic blocks

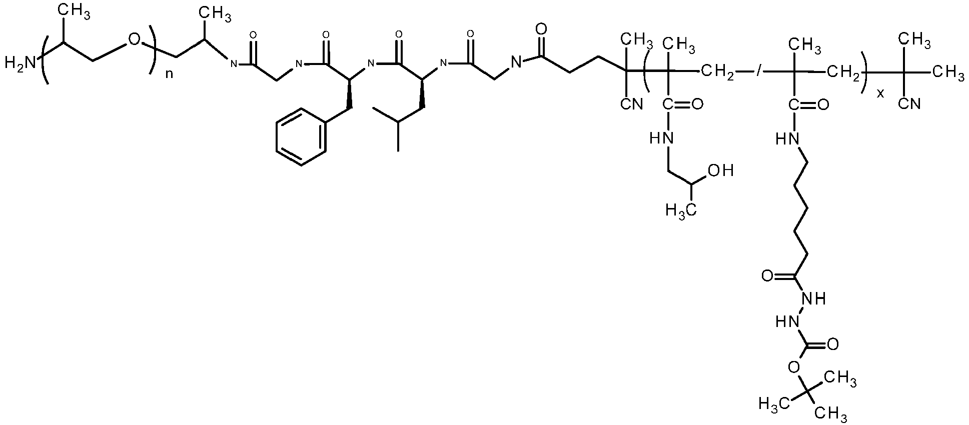

The semitelechelic copolymer raft-poly(HPMA-co-MA-AH-NHNH-Boc)-GFLG (23 mg; 2.15 x 10⁻³ mmol), prepared in Example 8, was dissolved in DMF and its solution was added to NH₂-PPO-NH₂ (M ~ 4,000 g/mol; 8.60 mg; 4.30 x 10⁻³ mmol -NH₂) and solid N-hydroxysuccinimide (NHS; 1.96 mg; 17 x 10⁻³ mmol). The reaction mixture was cooled using an ice water bath. To the reaction mixture was added a tenfold excess of *N,N-*diisopropylcarbodiimide (DIC) and the reaction mixture was allowed to react with stirring overnight. The product was purified on a column (LH-20, MeOH) and after evaporation of the solvent it was dried to constant weight. Characterization: *Mₙ* (SEC) = 15,500 g/mol; D = 1.11; conversion ~ 74% (23.6 mg); *Rₕ =* 19.2 nm.

### Example 18: Preparation of the A-L-B-L-A type amphiphilic triblock copolymer ([raft-poly(HPMA-co-MA-AH-NHNH-Boc)]₂-GFLG-PPO) with enzymatically cleavable bond between the hydrophilic and hydrophobic blocks

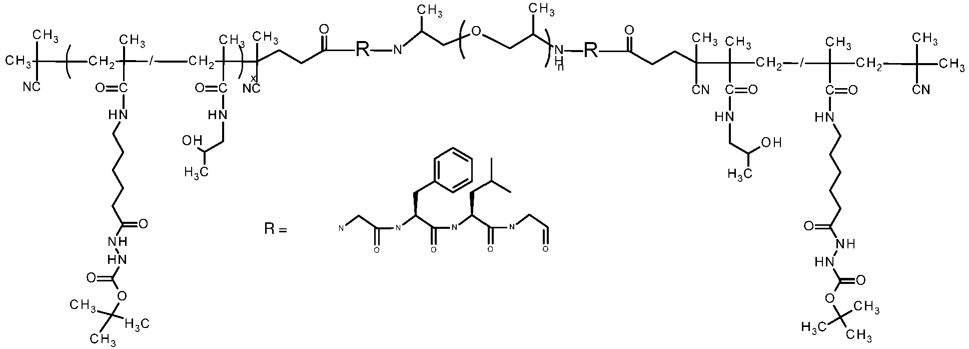

The triblock copolymer ([raft-poly(HPMA-co-MA-AH-NHNH-Boc)]₂-GFLG-PPO was prepared in the same manner as the diblock copolymer (Example 17), when the semitelechelic copolymer raft-poly(HPMA-co-MA-AH-NHNH-Boc)-GFLG, prepared according to Example 8, and NH₂-PPO-NH₂ were reacted in the presence of NHS and DIC in a molar ratio PHPMA: PPO 2: 1. Characterization: *Mₙ* (SEC) = 24,100 g/mol; D = 1.14; conversion ~ 70%; *R*ₕ = 17.2 nm.

### Example 19: Preparation of the B-L-A-L-B type amphiphilic triblock copolymer (PPO[raft-GFLG-poly(HPMA-co-MA-AH-NHNH-Boc)]-GFLG-PPO) with enzymatically cleavable bond between the hydrophilic and hydrophobic blocks

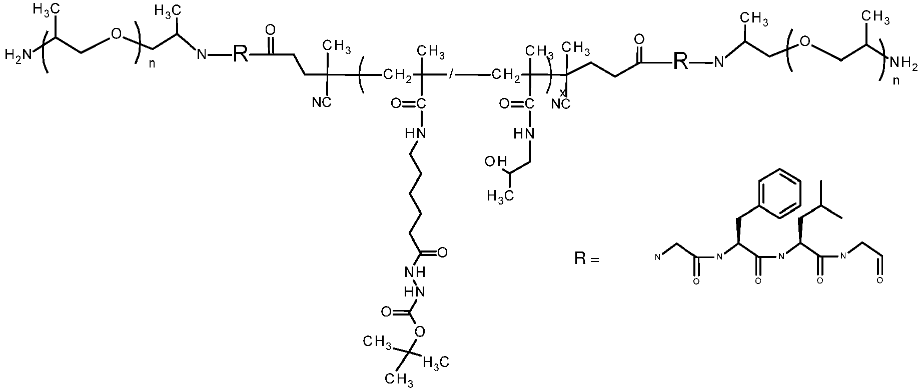

The triblock copolymer PPO[raft-GFLG-poly(HPMA-co-MA-AH-NHNH-Boc)]-GFLG-PPO was prepared by reaction of raft-GFLG-poly(HPMA-co-MA-AH-NHNH-Boc)-GFLG (Example 9) with NH₂-PPO-NH₂ in the presence of DIC and NHS in a similar manner as the triblock copolymer of Example 18, in a molar ratio PHPMA:PPO 1:2.

### Example 20: Synthesis of the A-B type reference non-degradable amphiphilic diblock copolymer ([raft-poly(HPAL4-co-MA-AH-NHNH-Boc)]-b-PPO-NH₂)

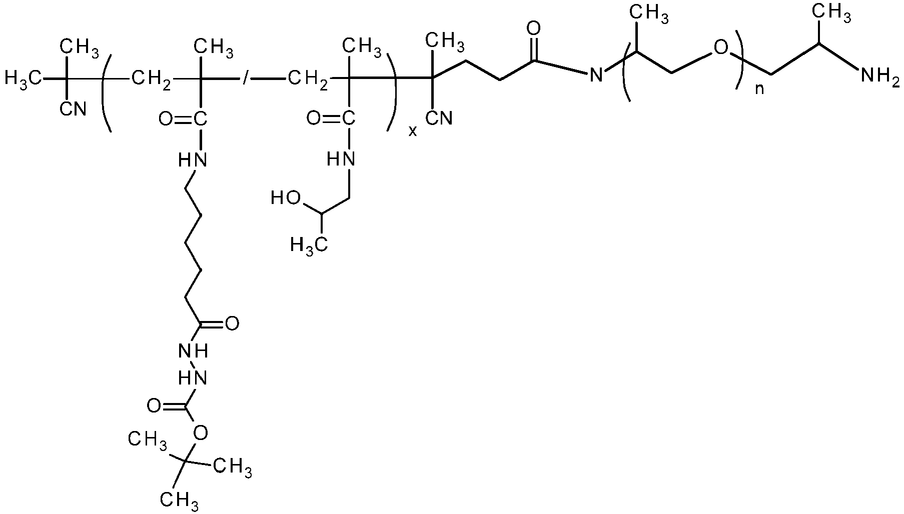

### [raft-poly(HPMA-co-MA-AH-NHNH-Boc)]-b-PPO-NH₂

In the synthesis of Boc-protected diblock copolymer [raft-poly(HPMA-co-MA-AH-NHNH-Boc)]-PPO-NH₂ we started from the commercially available poly(propylene oxide)-bis-2-aminopropyl ether (NH₂-PPO-NH₂; *M ~* 4,000 g/mol; 33.2 mg, 16.6 x 10⁻³ mmol -NH₂), which was dissolved in 1 mL of dimethylacetamide (DMA). Also the semitelechelic copolymer raft-poly(HPMA-co-MA-AH-NHNH-Boc)-TT, prepared in Example 4 (71 mg; 8.3 x 10⁻³ mmol, *Mₙ* ~ 7,614 g/mol, *F_{TT}* ~ 0.89) was dissolved in 1 mL of DMA. The solution of copolymer raft-poly(HPMA-co-MA-AH-NHNH-Boc)-TT was slowly added with stirring to a solution of NH₂-PPO-NH₂ and the mixture was reacted under stirring at room temperature overnight. After about 16 hours, the reaction mixture was diluted with methanol (MeOH) and purified by column chromatography over Sephadex LH-20 in MeOH with RI and UV detection (230 nm) and TLC. The purified product was then evaporated under vacuum to constant weight. Yield: 80 mg (77%). Characterization of the Boc-protected diblock polymer [raft-poly(HPMA-co-MA-AH-NHNH-Boc)]-PPO-NH₂: *M_{w}* ~ 15,230 g/mol, *Mₙ* - 12,950 g/mol (in the organic mobile phase), D ~ 1.18, *R*ₕ ~ 14 nm (in physiological saline or PBS).

### Example 21: Synthesis of the reference non-degradable amphiphilic triblock copolymer [raft-poly(HPMA-co-MA-AH-NHNH-Boc)]₂-PPO

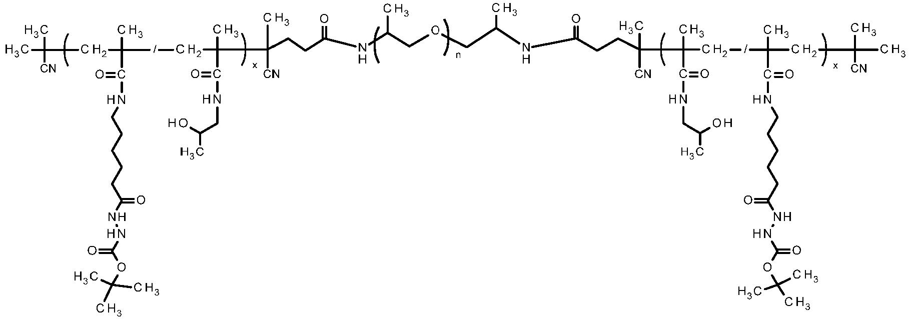

### [raft-poly(HPMA-co-MA-AH-NHNH-Boc)]₂-PPO

The A-B-A type amphiphilic triblock copolymer [raft-poly(HPMA-co-MA-AH-NHNH-Boc)]₂-PPO was prepared by the same procedure as its diblock analogue [raft-poly(HPMA-co-MA-AH NHNH-Boc)]-PPO-NH₂ of Example 20, i.e. by polycondensation of NH₂-PPO-NH₂ and semitelechelic copolymer raft-poly(HPMA-co-MA-AH-NHNH-Boc)-TT. NH₂-PPO-NH₂ (M ~ 4,000 g/mol; 28 mg; 14 x 10⁻³ mmol -NH₂) and raft-poly(HPMA-co-MA-AH-NHNH-Boc)-TT (115 mg; 14 x 10⁻³ mmol, *Mₙ* ~ 8,450 g/mol, F_{TT} ~ 0.98) were dissolved separately in DMA (1.575 mL). A solution of NH₂-PPO-NH₂ was slowly added dropwise under vigorous stirring to a solution of the semitelechelic copolymer raft-poly(HPMA-co-MA-AH-NHNH-Boc)-TT and the reaction mixture was allowed to react with constant stirring at room temperature. After 24 hours the reaction mixture was precipitated into diethyl ether (47 mL). The fine precipitate was centrifuged and the solvent was decanted. The polymer product was dried under vacuum to constant weight. Yield: 120 mg (84%). Characterization of the prepared Boc-protected triblock copolymer [raft-poly(HPMA-co-MA-AH-NHNH-Boc)]₂-PPO: *M_{w}* ~ 22,200 g/mol, *Mₙ* ~ 18,790 g/mol (in the organic mobile phase), *Ð* ~ 1.18, *R*ₕ ~ 15 nm (in physiological saline or PBS).

### Example 22: Synthesis of the amphiphilic diblock copolymer poly(HPMA-co-MA-GFLG-DOX)-GFLG-PPO-NH₂ with a drug bound via an enzymatically cleavable oligopeptide

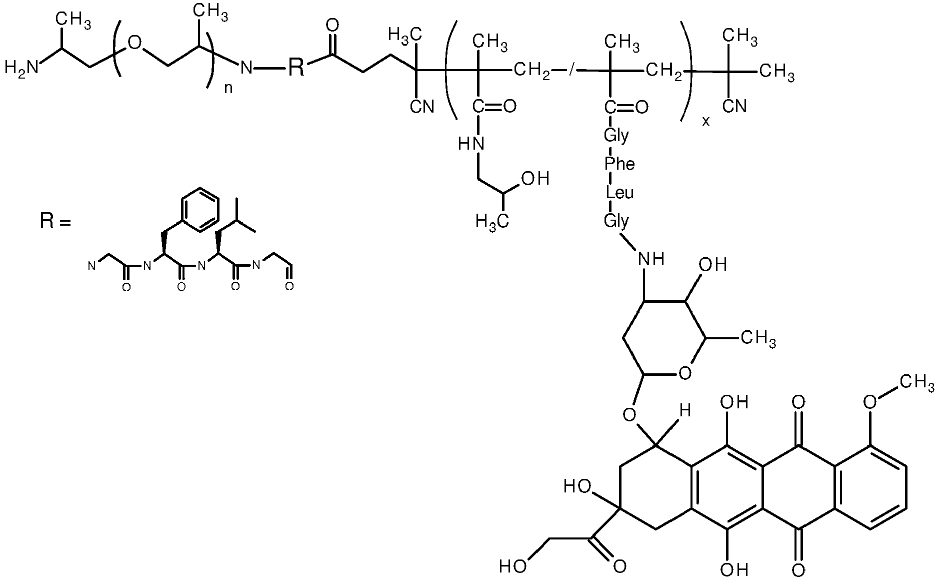

### poly(HPMA-co-MA-GFLG-DOX)-GFLG-PPO-NH₂

The semitelechelic copolymer poly(HPMA-co-MA-GFLG-DOX)-TT of Example 3 was first end-modified by tetrapeptide GFLG in the same manner as in Example 8 and the resulting semitelechelic copolymer poly(HPMA-co-MA-GFLG-DOX)-GFLG was used together with NH₂-PPO-NH₂, DIC and NHS in the synthesis of diblock copolymer poly(HPMA-co-MA-GFLG-DOX)-GFLG-PPO-NH₂, where we followed the same procedure as in Example 17. Yield: 78%. Characterization of the diblock polymer poly(HPMA-co-MA-GFLG-DOX)-GFLG-PPO-NH₂: *M_{w}* ~ 20,300 g/mol, *Mₙ* - 15,650 g/mol (in the organic mobile phase), *R*ₕ ~ 15 nm (in physiological saline or PBS).

### Example 23: Preparation of the A-L-B-L-A type triblock copolymer [raft-(polyHPMA)-GFLG]₂-PPO by RAFT polymerization using the polymer chain transfer agent PPO-GFLG-(CTA)₂

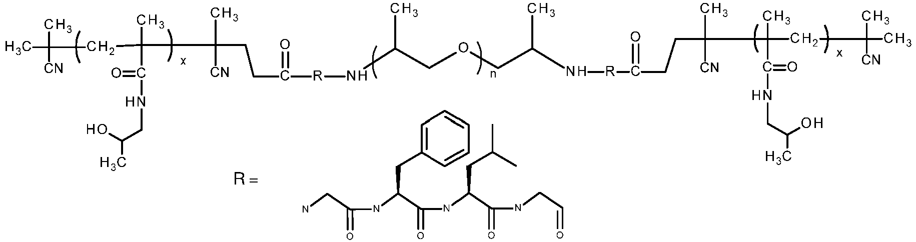

### [raft-(polyHPMA)-GFLG]₂-PPO

The A-L-B-L-A type triblock copolymer raft-(polyHPMA)₂-PPO was prepared by controlled radical RAFT polymerization using *PPO-(GFLG-CTA)₂* prepared in Example 1 as the chain transfer agent. The molar ratio of initiator/chain transfer agent/monomer was 1/2/150. HPMA (33.64 mg, 0.235 mmol) was dissolved in tert-butanol (0.232 mL), PPO (GFLG-CTA)₂ (10 mg; 4.35 x 10⁻³ mmol) and AIBN (0.36 mg; 2.18 x 10⁻³ mmol) were dissolved in DMSO (0.026 mL). These solutions were mixed together in a polymerization vial. The polymerization mixture was bubbled with argon for 10 min and then the vial was sealed. Copolymerization was carried out at 70°C for 16 hours. The product was isolated by precipitation into acetone/diethyl ether in the ratio 3/1. The fine precipitate was centrifuged, filtered off and dried to constant weight under vacuum. Reactive ω-terminal DTB groups were removed using AIBN. Characterization: *Mₙ* (SEC) ~ 14,600 g/mol (in the organic mobile phase), D ~ 1.13; conversion ~ 60% (26 mg); *R*ₕ ~ 14.5 nm (in physiological saline or PBS).

### Example 24: Preparation of the A-L-B-L-A type triblock copolymer [raft-poly(HPMA-coMA-AH-NHNH-Boc)-GFLG]₂-PPO by RAFT polymerization using the polymer chain transfer agent PPO- (GFLG-CTA)₂

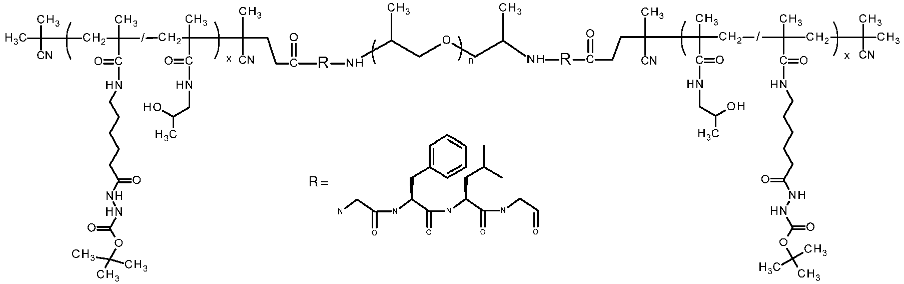

### [poly(HPMA-co-MA-AH-NHNH-Boc)-GFLG]₂-PPO

The triblock copolymer [raft-poly(HPMA-co-MA-AH-NHNH-Boc) -GFLG]₂-PPO was prepared similarly as the triblock copolymer [raft-(polyHPMA)-GFLG]₂-PPO of Example 23. The molar ratio of initiator/chain transfer agent/comonomers was 1/2/150. The ratio of comonomers HPMA/MA-AH-NHNH-Boc was 90/10 mol%. Reactive ω-terminal DTB groups were removed using AIBN.

Characterization: *Mₙ* (SEC) = 8,750 g/mol (in the organic mobile phase); D 1.56; conversion 50% (8.3 mg); *R*ₕ 14.0 nm (in physiological saline or PBS).

### Example 25: Preparation of a Bocprotected diblock copolymer containing in its structure biodegradable linkers Asp-Pro-Lys

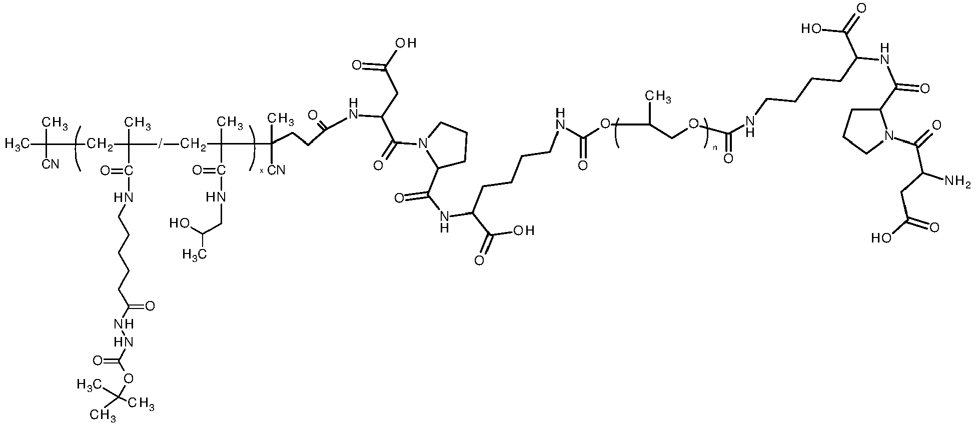

### Block copolymer raft-poly(HPMA-co-MA-AH-NHNH-Boc)-TT and PPO-(Asp-Pro-Lys)₂

The biodegradable diblock copolymer was prepared by polycondensation of the copolymer raft-poly(HPMA-co-MA-AH-NHNH-Boc)-TT and PPO-(Asp-Pro-Lys)₂ in a similar manner as the amphiphilic diblock copolymer [raft-poly(HPMA-co-MA-AH-NHNH-Boc)]-PPO-NH₂ (see Example 20). In a similar manner diblock copolymers containing other biodegradable oligopeptide linkers were prepared as listed in Example 13.

### Example 26: Preparation of the A-L-B-L-A type biodegradable triblock copolymer based on raft-poly(HPMA-co MA-AH-NHNH-Boc)-TT and PPO-(Asp-Pro-Lys)₂

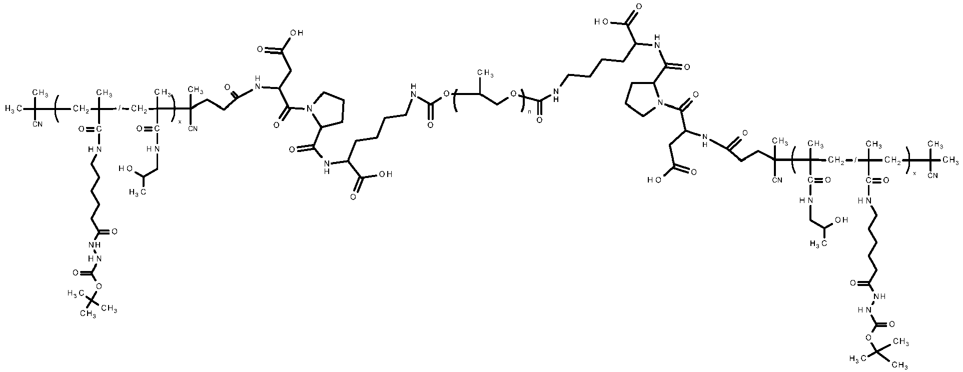

### Block copolymer raft-poly(HPMA-co MA-AH-NHNH-Boc)-TT and PPO-(Asp-Pro-Lys)₂

A biodegradable triblock copolymer based on raft-poly(HPMA-co-MA-AH-NHNH-Boc)-TT and PPO-(Asp-Pro-Lys)₂, containing a hydrolytically and enzymatically biodegradable peptide sequence Asp-Pro-Lys between the hydrophilic block A and hydrophobic block B, was prepared in a similar manner as the amphiphilic diblock copolymer raft-poly(HPMA-co-MA-AH-NHNH-Boc)-PPO (see Example 20), i.e. by polycondensation of the copolymer raft-poly(HPMA-co-MA-AH-NHNH-Boc)-TT and polymer PPO-(Asp-Pro-Lys)₂ in a molar ratio of 2:1. In a similar manner triblock copolymers containing other biodegradable oligopeptide linkers were prepared as listed in Example 13.

### Example 27: Removal of a Boc protecting group on hydrazide groups of the diblock polymer precursor [raft-poly(HPMA-co-MA-AH-NHNH-Boc)]GFLG-PPO-NH₂

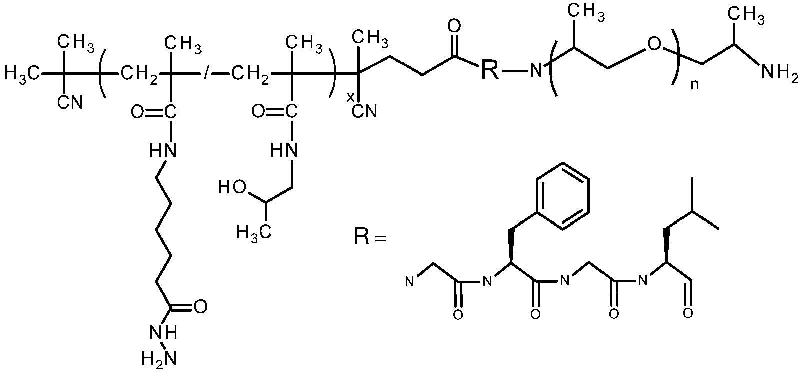

### [raft-poly(HPMA-co-MA-AH-NHNH₂)]-GFLG-PPO-NH₂

The diblock polymer precursor *[raft-poly(HPMA-co-MA-AH-NHNH₂)]-GFLG-PPO-NH₂* was prepared from the diblock copolymer [raft-poly(HPMA-co-MA-AH-NHNH-Boc)]-GFLG-PPO-NH₂ of Example 17 (50 mg) containing hydrazide groups protected by tert-butyloxycarbonyl group (Boc). The diblock copolymer [raft-poly(HPMA-co-MA-AH-NHNH-Boc)]-GFLG-PPO-NH₂ was dissolved in 1.08 mL of a mixture of trifluoroacetic acid (TFA)/triisopropylsilane (TIS)/water (95/2.5/2.5). Immediately after dissolving, the mixture was evaporated under vacuum to dryness. The residue was then dissolved in a borate buffer (pH 8.0; 200 µL) and the pH of the solution was adjusted by titration with a saturated NaOH solution to pH 6.5-8.0. The crude product was desalted and purified using a PD10 column (Sephadex G25) in water and lyophilized. Yield: 40 mg (78%). Characterization: *M_{w}* = 16,200 g/mol, *Mₙ =* 13,500 g/mol (in the organic mobile phase), *R*ₕ ~ 14.5 nm (in physiological saline or PBS).

In the same manner these protecting groups were removed also from polymer precursors prepared in Examples 15 to 21 and 24 to 26

### Example 28: Preparation of a diblock polymer conjugate with a drug bound via hydrolytically labile hydrazone linkage:

### (A) Diblock polymer conjugate [raft-poly(HPMA-co-MA-AH-NHN=DOX)]-GFLG-PPO-NH₂ with a hydrazone bound doxorubicin

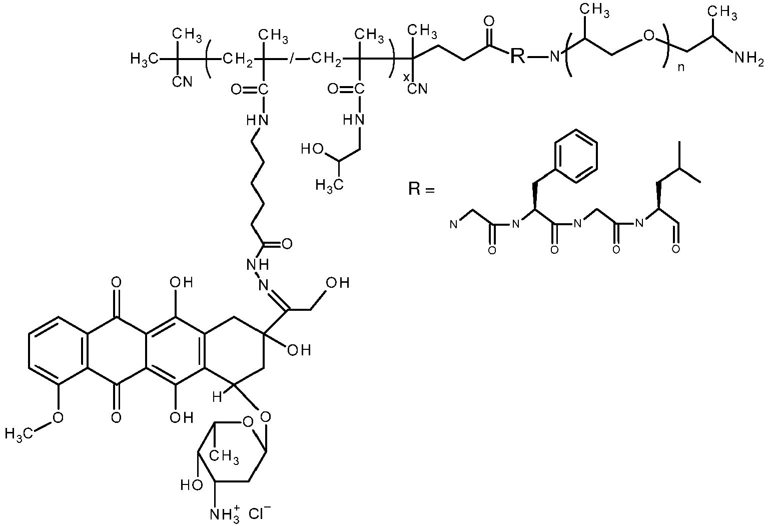

### [raft-poly(HPMA-co-MA-AH-NHN=DOX)]-GFLG-PPO-NH₂

The diblock polymer precursor [raft-poly(HPMA-co-MA-AH-NHNH₂)]-GFLG-PPO-NH₂, prepared in Example 27, with free hydrazide groups (120 mg) was dissolved in MeOH (960 µL). The polymer solution was added to the solid doxorubicin hydrochloride (DOX. HCl; 12.94 mg) and to the suspension was added 38.4 µL of concentrated acetic acid. The reaction mixture was shaken on a shaker in the dark at room temperature overnight. The reaction was monitored by TLC (CHCl₃/MeOH/acetic acid ~ 8/2/1). After 24 hours the reaction mixture was diluted with MeOH and the crude product was purified by column chromatography in MeOH over Sephadex LH-20. The polymer fraction was evaporated under vacuum to dryness and dried to constant weight. The content of bound doxorubicin was determined spectroscopically (λ ~ 488 nm, water, ε ~ 9,800 L x mol⁻¹ x cm⁻¹). The yield was 110.8 mg (83%), the content of drug on the polymer was 8.9 wt%. Characterization: *M_{w}* = 18,300 g/mol, *Mₙ =* 14,100 g/mol (in the organic mobile phase), *R*ₕ ~ 14.2 nm (in physiological saline or PBS). In a similar manner a diblock polymer conjugate with a drug pirarubicin or daunorubicin was prepared; the drug content for these conjugates was 9.9 wt% of pirarubicin or 9.5 wt% of daunorubicin.

Characterization of a conjugate with pirarubicin: *M*_{w} = 19,200 g/mol, *M*ₙ = 14,300 g/mol (in the organic mobile phase), *R*ₕ ~ 14.4 nm (in physiological saline or PBS). Characterization of a conjugate with daunorubicin: *M*_{w} = 18,800 g/mol, *Mₙ* = 14,500 g/mol (in the organic mobile phase), *R*ₕ ~ 13.9 nm (in physiological saline or PBS).

### (B) Diblock polymer conjugate with a hydrazone bound docetaxel derivative

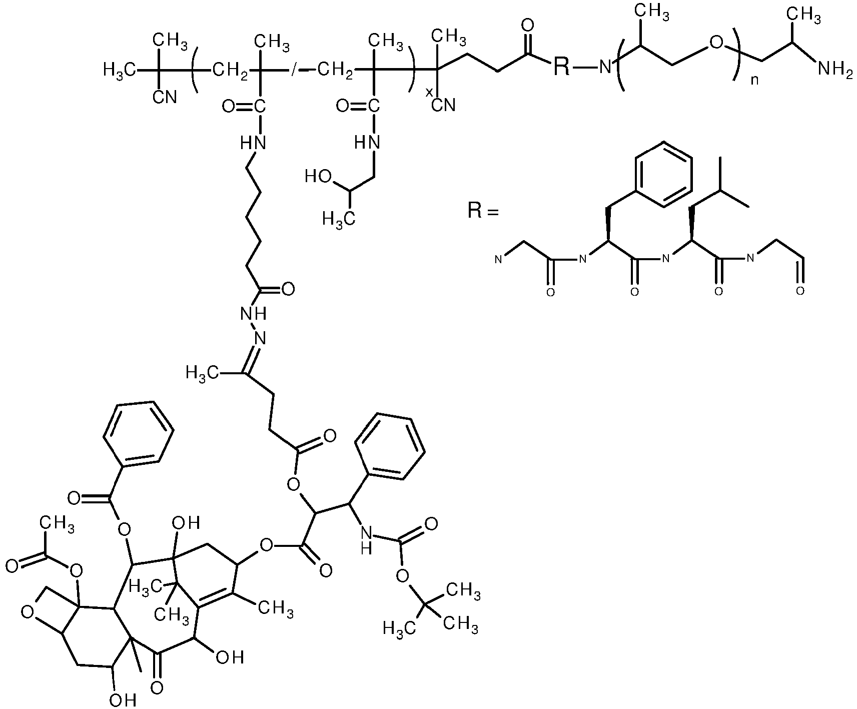

### [raft-poly(HPMA-co-MA-AH-NHN=DTX-LEV)]-GFLG-PPO-NH₂

The docetaxel derivatized by levulinic acid (DTX-LEV) was prepared by reacting docetaxel with levulinic acid in the presence of diisopropylcarbodiimide in DMSO. The diblock polymer precursor *[raft-poly(HPMA-co-MA-AH-NHNH₂)]-GFLG-PPO-NH₂* prepared in Example 27 (191.5 mg) and the derivative of drug DTX-LEV (22.9 mg) were dissolved in MeOH (172 µL) and the reaction mixture was immediately treated with concentrated acetic acid (70 µL). The reaction mixture was stirred for 2.5 hours. It was then diluted with MeOH and the product was purified by column chromatography in MeOH (Sephadex LH-20, MeOH; UV detection - 230 nm, RI]. The fraction of the polymer conjugate with DTX-LEV was collected, evaporated to dryness under vacuum and dried to constant weight. The yield was 172 mg (80%). Characterization: *M_{w}* = 17,500 g/mol, *Mₙ* = 13,600 g/mol (in the organic mobile phase), *Rₕ ~* 14 nm (in physiological saline or PBS). The content of the DTX-LEV derivative, after complete acidic hydrolysis (HCl with pH ~ 2.0) and extraction into chloroform, was determined by chromatography (HPLC) to be 9.0 wt%. In a similar manner also paclitaxel and larotaxel were derivatized and then both drugs were bound to a polymer carrier following the procedure as described above. Characterization of the conjugate with paclitaxel: *M*_{w} = 18,500 g/mol, *Mₙ* = 14,100 g/mol (in the organic mobile phase), *R*ₕ ~ 14.3 nm (in physiological saline or PBS). The content of paclitaxel-LEV derivative was 9.6 wt%. Characterization of the conjugate with larotaxel: *M_{w}* = 17,800 g/mol, Mₙ = 14,200 g/mol (in the organic mobile phase), *Rₕ ~* 14.1 nm (in physiological saline or PBS). The content of larotaxel-LEV derivative was 9.4 wt%. After removal of Boc protecting groups according to Example 27, it is possible using the same procedure as in Example 28 to bind the above-mentioned drugs even to polymer precursors prepared in Examples 15 to 21 and 24 to 26.

### Example 29: Preparation of a lyophilized form of polymer-drug conjugates

The block polymer-drug conjugates prepared as in Example 28 and the block polymer precursors without drugs were by lyophilization with additives converted into a lyophilized form, which facilitates conversion of a solid form of polymer drug into a solution and formation of nanoparticle micellar structures within 1 minute after adding the appropriate buffer or physiological saline.

The diblock polymer-drug conjugate prepared in Example 28 (100 mg; 8.7 wt% of DOX bound to polymer), NaH₂PO₄ x 2 H₂O (2.40 mg), Na₂HPO₄ x 2H₂O (9.54 mg) and lactose (332 mg) were dissolved in distilled water, the solution was frozen and lyophilized. The resulting lyophilizate (100 mg of the original polymer conjugate with the drug) was dissolved in 17.2 mL of an aqueous solution of NaCl with concentration of 9 g/L (physiological saline). Lyophilization cakes of polymer precursors and their conjugates with drugs were dissolved in physiological saline (0.9% of NaCl), which was prior to use filtered through Whatman membrane (PVDF) with a pore size of 0.20 µm.

### Example 30: Example of characterization of polymer precursors and conjugates

The prepared copolymers, polymer precursors and their conjugates with drugs were characterized by determination of weight and number average of molecular weights (*M_{w}*, *Mₙ)* and the respective polydispersity index (*Ð*) by means of the size exclusion chromatography (SEC) performed on a system equipped with an UV detector (Shimadzu, Japan), RI detector (Optilab REX, Wyatt Technology Corp., USA) and a multiangle light scattering detector (DAWN-Heleos II Wyatt Technology Corp., USA). Some systems were characterized using a FFF system (Eclipse 3+ module) equipped with DLS (DAWN 8+; Wyatt Technology Corp., USA), UV and RI (Optilab REX; Wyatt Technology Corp., USA) detection. For characterization, the TSK 3000 Super SW column was used in the case of SEC and the mixture of MeOH (80%) and 0.3M acetate buffer with pH 6.5 (20%) as a mobile phase, in the case of FFF the membrane of regenerated cellulose (10 kDa) was used for separation and water with NaN₃ as a mobile phase. The concentration of samples in all cases was 3 mg/mL.

The content of terminal -DTB, -DBCO,-TT groups and hydrazide groups copolymerized randomly along the polymer chain was determined spectrophotometrically. All measurements were performed on a UV-VIS spectrophotometer Specord 205 (Analytik Jena, Germany). The content of -TT groups was determined according to the literature (Šubr, V.; Koňák, Č.; Lagǎ, R.; Ulbrich, K. Biomacromolecules 2006, 7(1): 122-130) in methanol (*ε₃₀₅ =* 10,800 L . mol⁻¹ . cm⁻¹). The content of hydrazide groups (after deprotecting by a mixture of TFA (95%), triisopropylsilane (2.5%) and water (2,5%)) was determined according to the method described in Etrych, T.; Jelínková, M., Řihová, B. and Ulbrich, K., J. Control. Release 2001; 73: 89-102 (ε₅₀₀ = 17,200 L . mol⁻¹.cm⁻¹). The content of terminal -DBCO or -DTB groups was determined spectrophotometrically in MeOH (-DTB: *ε₃₀₂ =* 12,400 L . mol⁻¹.cm⁻¹, -DBCO: ε₃₀₂ = 13,000 L . mol⁻¹.cm⁻¹). The content of PDS groups was determined spectrophotometrically (*ε₃₄₃ =* 8,080 L . mol⁻¹ . cm⁻¹).

The sizes of nanoparticles, consisting of polymer precursors and conjugates with drugs in an aqueous medium, were determined by techniques of dynamic light scattering (Nano-ZS Zetasizer, Malvern) and static light scattering (ALV instrument, Germany) in aqueous buffer (PBS, pH 7.4). The static light scattering was measured in the range from 30 to 140° with steps of 10° at a concentration of 0.5 to 10 mg/mL and temperatures of 25°C and 37°C. The particle size distribution curve is shown in Figure 1.

### Example 31: Release of a drug

(A) Polymer conjugates with doxorubicin prepared according to Example 28A:
   The amount of doxorubicin released from polymer micelles was determined after incubation of polymer conjugates with DOX at 37°C in a phosphate buffer with pH 5.0 (0.1 M phosphate buffer containing 0.05 M of NaCl) modelling the environment in endosomes and lysosomes of tumour cells, and in a phosphate buffer with pH 7.4 (0.1 M phosphate buffer containing 0.05 M of NaCl), which models the bloodstream environment. A portion of the incubation solution was collected at predetermined time intervals and the amount of released DOX was determined by SEC (Shimadzu, Japan; isocratic flow rate 0.3 mL/min; mobile phase: MeOH (80%)/0.3 M acetate buffer with pH 6.5 (20%)) on TSK 3000 Super SW column.
   The amount of released DOX was calculated from the peak areas of free and bound DOX detected at a wavelength of 488 nm. While during incubation of polymer micelles at pH 5.0 there was a rapid release of DOX from the carrier (more than 70% of DOX released after 9 hours of incubation), during incubation of polymer conjugates in physiological environment (pH 7.4) the hydrolysis of hydrazone bond does practically not work and the drug is released only very slowly (up to 9% in 24 hours). The rate of DOX release from our prepared nanoparticulate systems in both environments (pH 5.0 and pH 7.4) is shown in Figure 2.
(B) Polymer conjugates with docetaxel derivative prepared according to Example 28B:
   The amount of docetaxel (DTX) or derivatives thereof released from polymer conjugates after their incubation in a phosphate buffer with pH 5.0 (0.1 M phosphate buffer containing 0.05 M of NaCl) modelling the intracellular environment and in a phosphate buffer with pH 7.4 (0.1 M phosphate buffer containing 0.05 M of NaCl) modelling the bloodstream environment was determined by HPLC. At predetermined time intervals 200 µL of incubation solution was collected and after the extraction of released drugs and derivatives thereof into chloroform their amounts were determined using an HPLC system (Shimadzu HPLC system equipped with a column with a reverse phase Chromolith Performance RP-18e (100 x 4.6 mm) and UV-VIS detector Shimadzu SPD-10AVvp (230 nm), eluent water-acetonitrile with gradient from 50 to 100 vol% of acetonitrile, flow rate of 0.5 mL x min⁻¹). After incubation of the conjugates (concentration 5 mg/mL) in a physiological environment at 37°C (phosphate buffer, pH 7.4) the drug DTX (or its derivative) is released significantly slower (Fig. 3) than in a slightly acidic environment at pH 5.0 modelling the environment in endosomes and lysosomes of tumour cells (Fig. 3). The disintegration of the drug derivative into free drug or direct cleaving of the drug from the polymer conjugate is presumably by the effect of enzymes, especially carboxyesterases.

### Example 32: The disintegration of PHPMA-L-PPO-based micelles

The disintegration of PHPMA-L-PPO-based micelles into polymer chains eliminable from the organism is possible in two ways: i) after the decrease in concentration of the sample below the critical micelle concentration (CMC), or ii) after the disintegration of biodegradable linkers between the copolymer blocks, or a combination of both procedures. The copolymer prepared according to Example 15, 17 and 25 was (after removal of Boc - protecting groups according to Example 27) dissolved in PBS buffer (0.15 mol.l⁻¹, pH 7.4) to a micellar solution at a concentration of 2 mg/mL and then diluted. The size of the micelles was monitored by a dynamic light scattering (QELS) technique. The CMC was measured with isothermal calorimetry (ITC). The CMC of deprotected diblock copolymers of Examples 15, 17 and 25 was set as 0.01 to 0.02 mg/mL. Below this concentration the micelles disintegrate into individual polymer chains eliminable from the organism. Enzymatic, hydrolytic and reductive degradations of deprotected PHPMA-L-PPO-based polymer diblock of Example 15, 16, 17, 18, 19, 22, 23, 24, 25 and 26 were studied in 0.1 M phosphate buffer (NaH₂PO₄/NaOH, pH = 6.0; 5 mM glutathion; 1 mM EDTA), containing the lysosomal enzyme cathepsin B, intracellular enzyme thioredoxin, i.e. in the environment modelling the intracellular environment of the tumour cells at substrate concentration of 10 mg/mL. Deprotected diblock or triblock copolymers of Examples above were dissolved in phosphate buffer at a concentration of 10 mg/mL and immediately before they are placed into a thermostat (temperature 37°C), an enzyme stock solution was added to get final concentration of cathepsin B or thioredoxin of 5.10⁻⁷ M. In cases of experiments which determine a simple hydrolytic or reductive degradation no enzyme was added to the solution. Aliquots (200 µL) of incubation solutions were collected at predetermined time intervals, which were desalted on PD-10 columns and lyophilized. The molecular weight of degradation products was measured in the same manner as described above (Example 30). Conjugates containing oligopeptide spacer Asp-Pro-Lys or GFLG and others were slowly degraded in a solution comprising cathepsin B (5.10⁻⁷ M).

After 48 hours of incubation with cathepsin B it was determined that 40% of polymer portions are in the form of soluble portions having a molecular weight below the threshold for renal filtration. Polymer conjugates with spacer Asp-Pro-Lys disintegrated even by simple hydrolysis, after 48 hours it was determined that 20% of the polymer portion is degraded. Similarly, reductive degradation using thioredoxin and glutathione proceeded very rapidly, after 10 hours these polymer conjugates degraded into individual polymer components. The aforementioned experiment confirmed the biodegradability of the block polymer conjugates into degradation products eliminable from the organism.

### Example 33: In vitro cytotoxic activity of the diblock and triblock polymers based on biodegradable PHPMA-L-PPO copolymers

The cytotoxic activity of biodegradable diblock and triblock polymer precursors based on PHPMA-L-PPO was studied in several cancer cell lines, particularly the neuroblastoma cell lines NB3, NB4 and the derived lines resistant to DOX (NB3/DOX and NB4/DOX), further in cell lines of T-cell lymphoma EL4, P388 lymphoma and derived resistant line P388/MDR. In *in vitro* experiments the cell viability was determined after 72 hours of incubation with various concentrations of the diblock and triblock polymer precursor using the AlamarBlue method. It was found that not even the concentration of 500 µg/mL of these polymer systems is toxic to the cells; it does not decrease their viability.

### Example 34: In vitro biological activity of the diblock and triblock polymers based on PHPMA-L-PPO as inhibitors of P-gp

The ability of biodegradable diblock and triblock polymers to inhibit transmembrane pumps, namely P-gps, which are one of the main causes of drug resistance, was demonstrated using a calcein assay. Experiments were performed in neuroblastoma cell lines NB3, NB4 and derived lines resistant to doxorubicin NB3/DOX and NB4/DOX and in lymphoma cell line P-388/MDR. In the aforementioned cell lines it was demonstrated that the drug resistance is caused by overexpression of P-gp.

In *in vitro* experiments the cells were incubated with various concentrations of biodegradable diblock and triblock polymers for various periods of time (0.5 hour, 2 hours and 6 hours or 4 hours, 8 hours and 24 hours), and then Calcein^{AM} was added thereto for 20 minutes, which is cleaved in the cells into fluorescence-activated Calcein (excitation 485 nm/emission 530 nm). At the same time, Calcein is removed from the cells with increased activity of P-gp. The extent of ability to inhibit P-gp was determined as the fluorescent activity of Calcein as compared to control (10 mM Cyclosporin A - CsA, low molecular weight inhibitor of P-gp). The significant ability to inhibit P-gp was detected in all studied DOX resistant lines, namely NB3/DOX (Fig. 4), NB4/DOX (Fig. 5) and P388/MDR (Fig. 6). For all cell lines, the highest rate of P-gp inhibition was observed for the highest concentrations 250 and 500 µg/mL and decreased with decreasing concentration of the polymers used. Degradable polymer diblocks PPO-L-pHPMA showed in all studied lines significantly higher inhibition ability than the non-degradable PPO-pHPMA system (prepared in Example 20). For parental cell lines NB3, NB4 and P388 no determinant change was detected, which is consistent with the very low expression of P-gp in these cell lines. The studied concentrations of polymers ranging from 30 to 250 µg/mL are fully consistent with the expected concentrations of polymer systems used in the treatment of solid tumours exhibiting a multiple drug resistance. An increase of P-gp inhibition was observed in the use of all the degradable polymer conjugates. Given the rate of disintegration of the amphiphilic polymer systems, the highest P-gp inhibition was observed for systems with a disulphide linker, where the degradation rate was the highest.

### Example 35: Comparison of the in vitro biological activity of diblock and triblock conjugates - influencing the cytotoxic effect

The effect was monitored of P-gp inhibition in the cells of neuroblastoma lines using the diblock copolymer of pUPMA-L-PPO of Example 15 (where L is a disulphide linker) and triblock copolymer (PHPMA-L)₂-PPO (where L is Asp-Pro-Lys) prepared in Example 26, with free hydrazide groups removed as described in Example 27, on the change of IC₅₀ of drugs: i) DOX; ii) polymer doxorubicin PHPMA-DOX (the linear hydrazone conjugate with DOX based on PHPMA); iii) a diblock polymer conjugate of Example 28 (DB-DOX). In *in vitro* experiments the cells were first incubated for 1 hour with/without a diblock or triblock copolymer at a concentration 250 µg/mL and thereto were added the above mentioned drugs in a concentration series. After 72 hours of incubation the metabolic activity of cells was determined using the AlamarBlue method and from it the IC₅₀ of individual drugs. The results are shown in Table 5. An experiment was performed with non-degradable diblock polymers PHPMA-PPO (Example 20) and triblock polymers (PHPMA)₂-PPO (Example 21) as a control.

**Table 5: Increased cytotoxicity of drugs: doxorubicin (DOX), the linear hydrazone conjugate with DOX based on PHPMA (PHPMA-DOX) and the A-L-B type diblock conjugate of Example 28 (DB-DOX) caused by blocking the P-gp transmembrane pumps in neuroblastoma cell lines resistant to doxorubicin: NB3/DOX and NB4/DOX, measured by the AlamarBlue method. Table 5 shows the IC₅₀ values, i.e. concentrations in µg/mL at which the viability of one half of test cells is reduced.**

| IC₅₀ after 72 hours of incubation | NB3/DOX | NB4/DOX |
|---|---|---|
| DOX | 0.215 ± 0.004 | 2.61 ± 0.17 |
| DOX + PHPMA-PPO (250 ug/mL) | 0.020 ± 0.001 | 0.42 ± 0.03 |
| DOX + PHPMA-L-PPO (250 ug/mL) | 0.010 ± 0.001 | 0.25 ± 0.02 |

| IC₅₀ after 72 hours of incubation | NB3/DOX | |
|---|---|---|
| DOX | 0.168 ± 0.005 | |
| DOX + (PHPMA)₂-PPO (250 ug/mL) | 0.032 ± 0.003 | |
| DOX + (PHPMA-L)₂-PPO (250 ug/mL) | 0.020 ± 0.002 | |
| | | |

| IC₅₀ after 72 hours of incubation | NB3/DOX | NB4/DOX |
|---|---|---|
| PHPMA-DOX | 2.596 ± 0.132 | 35.00 ± 2.79 |
| PHPMA-DOX + PHPMA-PPO (250 ug/mL) | 0.340 ± 0.006 | 5.60 ± 0.32 |
| PHPMA-DOX + *PHPMA-L-PPO* (250 ug/mL) | 0.180 ± 0.004 | 2.90 ± 0.21 |

| IC₅₀ after 72 hours of incubation | NB3/DOX | |
|---|---|---|
| PHPMA-DOX | 3.338 ± 0.110 | |
| PHPMA-DOX + (PHPMA)₂-PPO (250 ug/mL) | 0.521 ± 0.006 | |
| PHPMA-DOX + (PHPMA-L)₂-PPO (250 ug/mL) | 0.385 ± 0.009 | |
| | | |

| IC₅₀ after 72 hours of incubation | NB3/DOX | NB4/DOX |
|---|---|---|
| DB-DOX | 0.919 ± 0.020 | 7.07 ± 0.11 |
| DB-DOX + PHPMA-PPO (250 ug/mL) | 0.152 ± 0.007 | 3.92 ± 0.13 |
| DB-DOX + PHPMA-L-PPO | 0.101 ± 0.005 | 2.85 ± 0.16 |
| (250 ug/mL) | | |

Table 5 shows that both PHPMA-L-PPO (copolymer of Example 15) and (PHPMA-L)₂-PPO (copolymer of Example 26 with free hydrazide groups) at a concentration of 250 µg/mL ensured the increase of cytotoxicity, i.e. reduction of IC₅₀ value of the polymer conjugate PHPMA-DOX and free DOX by more than one order of magnitude. A slightly higher effect was observed in PHPMA-L-PPO than in (PHPMA-L)₂-PPO. For PHPMA-L-PPO higher effect was observed on tumour resistant cell line NB3/DOX that expresses higher amounts of P-gp transmembrane pump. From the aforementioned results it can be concluded that the inhibitory effect on resistant tumour cells will be higher, the higher the level of P-gp transmembrane pumps is. When comparing degradable systems PHPMA-L-PPO and (PHPMA-L)₂-PPO with non-degradable systems PHPMA-PPO and (PHPMA)₂-PPO, the degradable systems seem approximately twice as good, which is fully in accordance with the results of calcein assay. A similar increase in cytotoxicity was observed even when other degradable conjugates were used. Given the rate of disintegration of the amphiphilic polymer systems, the highest inhibitory ability against P-gp was observed for systems with a disulphide linker, where the degradation rate was the highest.

The actual diblock polymer conjugate of Example 28 (DB-DOX) exhibits a 3 to 5 times higher cytotoxicity in both resistant neuroblastoma cell lines than PHPMA-DOX, a linear hydrazone conjugate with DOX (concentration of DB carrier at IC₅₀ is approximately 10 µg/mL). Adding PHPMA-L-PPO (copolymer of Example 15) to the DB-DOX conjugate of Example 28 increased the cytotoxicity of DB-DOX less than in the case of PHPMA-DOX or free DOX. The effect of PHPMA-L-PPO is already evident in the biological *in vitro* activity of DB-DOX itself and adding of PHPMA-L-PPO (copolymer of Example 15) has only a supplementary effect.

It was demonstrated that the degradable diblock polymer PHPMA-L-PPO is capable of increasing the cytotoxic effect in selected resistant tumour cell lines by more than one order of magnitude. This effect is shown for both other long-circulating polymer drugs (e.g. PHPMA-DOX) and classical low molecular weight drug DOX. The actual polymer conjugate with a drug based on block copolymers PHPMA-L-PPO of Example 28 (DB-DOX) shows significantly higher cytostatic effect on resistant tumour cell lines than similar PHPMA-based polymer systems.

### Example 36: Cytostatic in vitro activity of the biodegradable diblock micellar doxorubicin conjugate of Example 28A (DB-DOX) when acting upon the tumour cell lines

To demonstrate the cytostatic activity of the micellar diblock polymer conjugate carrying doxorubicin of Example 28A (DB-DOX) the cells were used of several stable tumour cell lines having different origins. These were murine tumour cell lines (CT26 - colon carcinoma cell line, 4T1 - mammary carcinoma cell line, both originating from the BALB/c inbred strain of mice, followed by EL4.IL-2-line of T-cell lymphoma originating from the C57BL/6 inbred strain of mice) and FaDu - a human line of squamous cell carcinoma of the head and neck. The cells were cultured in 96-well culture plates with different concentrations of DB-DOX for 72 hours. The cell proliferation was then determined using the standard method of incorporating ³H-thymidine which was added for the last 6 hours of culture. Subsequently, the cultures were processed using the Tomtec Mach III harvester and, after drying, the radioactivity of samples obtained was measured by the beta-scintillation counter Microbeta Trilux (Wallac) using a solid scintillant (MeltiLex; Perkin Elmer). Test results are expressed as IC₅₀ value equal to the concentration of DB-DOX (indicated in terms of doxorubicin content in the conjugate), which is required for induction of just 50% inhibition of growth (proliferation) of the tumour cells in question (see Table 6). The cytostatic activity of DB-Dox was compared with the activity of other micellar HPMA-based conjugate with doxorubicin (HPMA copolymer with cholesterol units self-assembling into micellar structures, for reference see Chytil P., Etrych T., Šírová M., Mrkvan T., Řihová B., Ulbrich K., *J. Controlled Release 127, 121-130, 2008),* and further with the activity of the linear HPMA copolymer with doxorubicin (water-soluble HPMA copolymer, for reference see Etrych T., Jelínková M., Říhová B., Ulbrich K, J. Controlled Release 73, 89-102,2001*),* and with a free drug.

**Table 6: Cytostatic effectiveness of DB-DOX of Example 28A, other HPMA-based conjugates and free doxorubicin in four selected tumour cell lines. The activity of conjugates was determined by ³H-thymidine incorporation after 72 hours of incubation of cells with various concentrations of drugs. The IC₅₀ values are given in ng of doxorubicin/mL and were calculated as a mean of several independent determinations.**

| | CT26 | 4T1 | EL4.IL-2 | FaDu |
|---|---|---|---|---|
| DB-DOX (conjugate of Example 28A) | 105.1 | 58.4 | 98.6 | 26.7 |
| Micellar conjugate | 117.6 | 56.1 | 125.4 | 31.7 |
| Linear conjugate | 232.3 | 101.6 | 195.1 | 63 |
| Doxorubicin | 42.5 | 14.3 | 12.2 | 4.8 |

For all lines tested, namely those sensitive to doxorubicin (FaDu) or slightly sensitive (CT26), it can be seen that the cytostatic activity of DB-DOX (conjugate according to Example 28A) is equal to or even slightly higher than that of another type of micellar conjugate based on HPMA. The linear conjugate with doxorubicin (DOX-PHPMA) showed activity in all lines substantially lower than DB-DOX (conjugate of Example 28). For the line having a high degree of natural resistance to a cytostatic agent (CT26) the cytostatic effect of doxorubicin decreased by binding the drug to a diblock carrier only 2.6 times, while for more drug sensitive lines it was 6 to 8.6 times (EL4.IL-2 and FaDu) (see Table 6). In the entire range of concentrations used the amount of diblock polymer system was lower than concentrations demonstrated as functional in the ABC transporter blockade, see above. The diblock micellar conjugate with doxorubicin (conjugate of Example 28) shows a significant cytostatic activity against tumour cell lines of various origins *in vitro.*

### Example 37: In vivo biological activity of micellar diblock conjugate with doxorubicin of Example 28A (DB-DOX) in the treatment of murine T-cell lymphoma EL4

The *in vivo* antitumour effect of micellar diblock polymer conjugate of Example 28 carrying doxorubicin (DOX-DB) was studied on a model of murine T-cell lymphoma EL4 in the C57BL/6 inbred strains of mice. The conjugate was administered to mice intravenously (i.v.) in a single dose equivalent to 7.5 mg of doxorubicin/kg. The drug was injected 8 days after subcutaneous administration of 1 x 10⁵ EL4 tumour cells, at a time when tumours are developed and the tumour bed size is about 6-8 mm in diameter. For comparing the therapeutic effect of the diblock copolymer of Example 28A (DB-DOX), we administered the linear HPMA copolymer with doxorubicin (PHPMA-DOX) at the same dose. Both the micellar diblock conjugate of Example 28A (DB-DOX) and linear conjugate (PHPMA-DOX) carried doxorubicin linked via a hydrazone bond. Further, the free doxorubicin (DOX) was used for treatment, injected at a total dose of 10 mg/kg, which, due to the different pharmacokinetics of a low molecular weight drug and the associated danger of high systemic toxicity, was injected in two partial intravenous doses on the 8^{th} and 12^{th} day after transplantation of tumour cells (each dose of 5 mg of doxorubicin).

Mice in the group treated with the diblock conjugate of Example 28A (DB-DOX) showed in comparison to untreated controls a tumour growth stagnation within three days and a significant reduction in growth between the third and sixth day after administration of the drug. A similar stagnation of tumour growth showed also the linear conjugate treated group (PHPMA-DOX), within six days there was a reduction in tumours, but was less pronounced than in the group treated with DB-DOX (conjugate of Example 28). In contrast, in the group treated with free doxorubicin (DOX) the average tumour growth was slower than in the group of untreated mice, but showed a steady progression. As early as the eighth day after drug administration the first mouse with complete tumour regression appeared in the group treated with DB-DOX, 13 days after drug administration there were 4 mice in the group (out of 8) without apparent tumour at the place of administration, while in other mice the tumours gradually grow, however their size was always significantly lower than in untreated controls at the same time. The reappearance of a small tumour bed in the original localization occurred in one out of 4 mice in which the complete tumour regression has been previously observed, and this was very late (after the 40^{th} day of experiment, that is 32 days after drug administration). The mean survival of untreated mice is 29 days (SD 2.0 days, mean survival time 29 days). The diblock conjugate DB-DOX (conjugate of Example 28) thus eventually induced complete cure in 3 out of 8 subjects (37.5%) and the survival of other mice was significantly prolonged compared to untreated controls (see Table 7). Prolongation of survival in the group treated with DB-DOX (conjugate of Example 28A) is statistically significant and the median survival is 47.5 days versus 29 in the group of untreated mice. The linear conjugate with doxorubicin (PHPMA-DOX) cured only one mouse (12.5%), whereas the free doxorubicin (DOX) cured none. The average tumour growth is shown in Figure 7a, the survival of mice after treatment is shown in Table 7 and Figure 7b.

**Table 7: Survival of C57B/6 mice inoculated with murine EL4 lymphoma, treated with micellar diblock conjugate DB-Dox according to Example 28 after i.v. administration of a single dose of 7.5 mg/kg or linear conjugate with doxorubicin (PHPMA-DOX) at the same dose or free doxorubicin (DOX) administered in two doses (2 x 5 mg/kg). Days mean the death of animals after administration of tumour cells (1 x 10⁵ cells EL4 s.c. on day 0), LTS ("long term survivor") means long-term surviving mice without evidence of tumour (at least until day 70 after administration of tumour cells). The last line contains information about the median survival in groups.**

| DB-DOX | Linear conjugate | Doxorubicin | Untreated controls |
|---|---|---|---|
| 1 x 7.5 mg DOX eq./kg | 1 x 7.5 mg DOX eq./kg | 2 x 5 mg DOX/kg | 0 mg DOX/kg |
| 37 | 30 | 35 | 27 |
| 39 | 34 | 38 | 27 |
| 42 | 35 | 39 | 27 |
| 42 | 37 | 42 | 28 |
| 60 | 42 | 53 | 30 |
| LTS | 44 | 53 | 30 |
| LTS | 45 | -- | 31 |
| LTS | LTS | -- | 32 |
| 47.5 | 39.5 | 40.5 | 29 |

Administration of DB-DOX (conjugate according to Example 28A) did not induce in the treated mice any observable signs of systemic toxicity (e.g. reduced body weight, bristly hair, hunched position, eating disorders). Without risk of systemic toxicity even higher doses of this drug may be administered. An important feature of treatment with the diblock conjugate according to Example 28A (DB-DOX) is the establishment of a resistance against tumour, which is tumour- specific, long-term and mediated by antitumour immune mechanisms. It was demonstrated by retransplantation of cured mice (LTS) with the same dose of live EL4 tumour cells that was used to induce the disease, but with leaving these mice without any treatment. Resistant individuals after this retransplantation developed no tumour and survive in the long term. Mice cured in the described experiment (LTS) were retransplanted as late as 119 days after administration of the first tumour, and of these three mice two were resistant. The micellar conjugate according to Example 28A (DB-DOX) demonstrated high activity as a delivery system for a cytotoxic drug (doxorubicin) in the treatment of the experimental mouse lymphoma.

### Example 38: Drug accumulation in solid tumours and time of circulation in the bloodstream

The drug accumulation in solid tumours and time of its circulation in the blood were determined in mice inoculated with T-cell lymphoma EL4. At predetermined time intervals, blood was collected from mice along with tumour samples. These samples were homogenized and analyzed for drug content. The drug content was determined after acid hydrolysis in 1 M HCl at 50°C when DOX decomposes into aglycone, which is then shaken into an organic solvent immiscible with water and assayed by HPLC (Shimadzu HPLC system equipped with a reverse phase column Chromolith Performance RP-18e (100 x 4.6 mm) and UV-VIS detector Shimadzu SPD-10AVvp (230 nm); eluent: water-acetonitrile with the gradient of 50-100 vol% of acetonitrile, flow rate 0.5 mL · min⁻¹). After administration of the diblock conjugate based on PHPMA-L-PPO with DOX attached via a hydrazone bond (DB-DOX, i.e. the conjugate according to Example 28) 4 times higher amount of drug was determined in the tumour tissue than with the linear PHPMA conjugate (PHPMA-DOX), see Figure 8. It was also found that the accumulation in the tumour tissue is more than 20-fold as compared with the drug accumulation following administration of a free drug at the maximum tolerated dose. Also, in determining the time of circulation in blood it was found that the drug bound to the diblock PHPMA-L-PPO polymer system (conjugate according to Example 28) circulates in the bloodstream considerably longer than in the case of linear PHPMA conjugate (PHPMA-DOX) and the free drug (DOX) (see Fig. 9). Both experiments demonstrated the ability of block PHPMA-PPO-based copolymers to be highly effective delivery systems for pharmaceuticals having significantly better pharmacokinetic parameters.

## Claims

1. A block copolymer for overcoming drug resistance of tumours to chemotherapy, comprising at least one block A and at least one block B, wherein at least one block A and at least one block B are connected to each other with a linker L, which is hydrolytically, enzymatically and/or reductively biodegradable and is selected from the group comprising oligopeptides with two to five amino acids
and/or a disulfide bond, and wherein the hydrophilic block A is formed by poly(N-(2-hydroxypropyl) methacrylamide), eventually containing up to 20 mol% of monomer units of general formula (I), 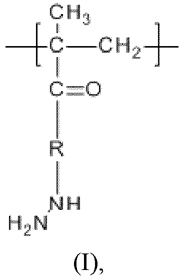
where R is aminoacyl selected from the group consisting of glycyl, glycylglycyl, β-alanyl, 6-aminohexanoyl, 4-aminobenzoyl, acyles derived from oligopeptides of two to five amino acids, especially GlyPheGly, GlyLeuGly, GlyLeuPheGly and GlyPheLeuGly;
and/or up to 15 mol% of monomer units of general formula (II), 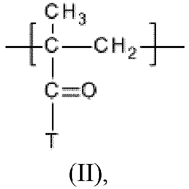
where T is oligopeptide attached to the monomer unit via an amide bond, wherein the oligopeptide is selected from the group consisting of the oligopeptides of two to five amino acids, preferably the oligopeptide is GlyPheGly, GlyLeuGly, GlyLeuPheGly, GlyPheLeuGly, AspProLys, AspProSer, and AspProGly;
and the hydrophobic block B is formed by a polypropylene oxide;
wherein the block A has its molecular weight *Mₙ* in the range of from 4,000 to 60,000 g/mol, and the block B has its molecular weight *Mₙ* in the range of from 1,000 to 17,600 g/mol;
and wherein the molecular weight of the block copolymer is in the range of from 5,000 to 95,000 g/mol;
wherein the block copolymer may further comprise terminal groups, wherein the terminal group of block A is 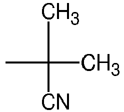 and the terminal group of block B is selected from the group consisting of -NH₂, 2-aminopropyl, dibenzocyclooctyle, and an oligopeptide of two to five amino acids, connected to PPO via an amide bond.

2. The block copolymer according to claim 1, **characterized in that** blocks A and B are arranged in a linear or branched topology; preferably blocks A and B are arranged in the order of A-L-B or A-L-B-L-A or B-L-A-L-B, or according to a general formula (III)

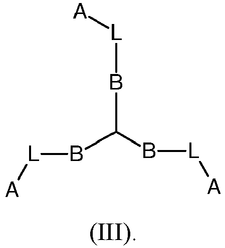

3. The block copolymer according to claim 1 or 2, **characterized in that** the linker L is oligopeptide selected from the group consisting of AspProLys, AspProLysProAsp, GlyLeuGly, GlyPheGly, GlyPheLeuGly, GlyLeuPheGly and/or a disulfide bond.

4. A polymer conjugate, which comprises the block copolymer according to the claim 1, 2 or 3, which further contains from 1 to 25 wt% of a low molecular weight drug covalently bound to the monomer unit of the general formula (I) and/or to the monomer unit of the general formula (II) of the block A by a hydrolytically and/or enzymatically cleavable covalent bond, preferably the drug is a low molecular weight cytostatic, most preferably the low molecular weight cytostatic is selected from the group comprising doxorubicin, docetaxel, paclitaxel, pirarubicin, mitomycin C, daunorubicin, larotaxel.

5. The polymer conjugate according to claim 4, **characterized in that** the molecule of the low molecular weight drug is bound to the monomer unit of the general formula (I) and/or to the monomer unit of the general formula (II) of the block A of the polymer conjugate by a hydrazone or a peptide bond.

6. The block copolymer according to any one of the claims 1 to 3 and/or the polymer conjugate according to any one of the claims 4 and 5, **characterized in that** it is in an aqueous medium in the form of a micelle, wherein the aqueous medium comprises water, saline, body fluids, particularly blood and blood plasma.

7. A method of preparation of the block copolymer according to the claim 1, 2 or 3, wherein the said method comprises the following steps:
- a step of radical polymerization of N-(2-hydroxypropyl)methacrylamide, eventually containing up to 20 mol% of the monomer of the general formula (I), where R is aminoacyl selected from the group consisting of glycyl, glycylglycyl, beta-alanyl, 6-aminohexanoyl, 4-aminobenzoyl, acyles derived from oligopeptides of two to five amino acids, particularly GlyPheGly, GlyLeuGly, GlyLeuPheGly and GlyPheLeuGly, protected on hydrazide groups by tert-butyloxycarbonyl (Boc) groups, and/or optionally containing up to 15 mol% of the monomer of the general formula (II), where T is oligopeptide attached to the monomer unit through an amide bond, wherein the oligopeptide is selected from the group consisting of oligopeptides of two to five amino acids, preferably the oligopeptide is GlyPheGly, GlyLeuGly, GlyLeuPheGly, GlyPheLeuGly, AspProLys , AspProSer and AspProGly;
at a temperature ranging from 40 to 100°C, preferably from 50 to 80°C, and in a solvent preferably selected from the group comprising of DMSO, aqueous buffers, dimethylacetamide, dimethylformamide, methanol, ethanol, dioxane, tetrahydrofuran, propanol, tert-butanol or mixtures thereof, initiated with an initiator, preferably selected from the group comprising ABIK-TT and ABIK-N₃, where ABIK-TT is 2-[(Z)-[1-cyano-1-methyl-4-oxo-4-(2-thioxothiazolidin-3-yl)butyl]azo]-2-methyl-5-oxo-5-(2-thioxothiazolidin-3-yl)pentanenitrile and ABIK-N₃ is N-(3-azidopropyl)-4-[(Z)-[4-(3-azidopropylamino)-1-cyano-1-methyl-4-oxo-butyl]azo]-4-cyano-pentanamide, optionally in the presence of a chain transfer agent, preferably selected from the group of CTA-TT and CTA-N₃, where CTA-TT is [1-cyano-1-methyl-4-oxo-4-(2-thioxothiazolidin-3-yl)butyl]benzencarbodithioate and CTA-N₃ is [4-(3-azidopropylamino)-1-cyano-1-methyl-4-oxo-butyl]benzencarbodithioate, to form the hydrophilic block A as a semitelechelic hydrophilic polymer block A, preferably with a terminal TT or N₃ group; wherein the terminal TT or N₃ group of the resulting semitelechelic hydrophilic polymer block A may further react with aminoethylpyridyldisulfide to give semitelechelic hydrophilic polymer block A with a terminal aminoethylpyridyldisulfide (PDS) group, or with a peptide of two to five amino acids to give a semitelechelic hydrophilic polymer block A with a terminal peptide chain, as a precursor of the linker L;
- a step in which the semitelechelic hydrophilic polymer block A from the previous step reacts with the semitelechelic or telechelic hydrophobic polymer block B of general formula Y-PPO-Y, Y-L-PPO-L-Y or PPO-L-Y or 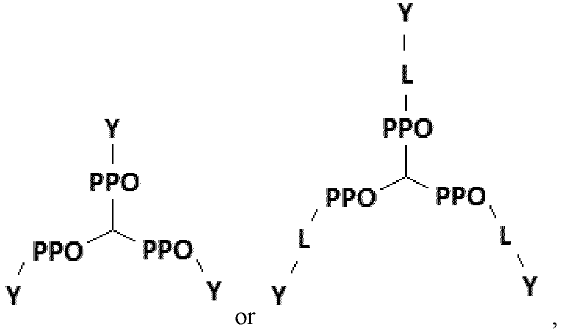 wherein Y is -NH₂, oligopeptide of two to five amino acids, succinimidyl carbonate or dibenzocyclooctyne group, and wherein L is oligopeptide of two to five amino acids or an -S-S- bond, and wherein L may be attached to the block B and group Y by an amide bond or as a part of a linking chain of general formula (VIII) 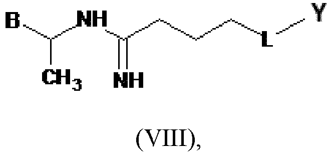 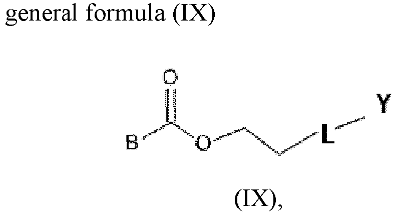 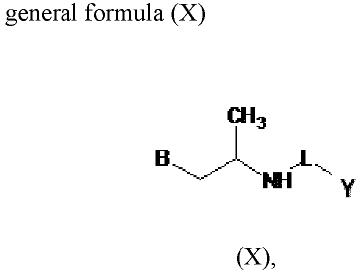 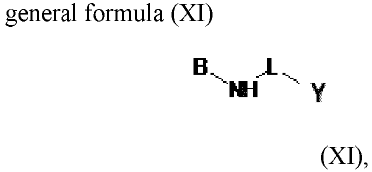 or with the chain transfer agent PPO-(L-CTA)₂, where L is peptide of two to five amino acids or -S-S- bond,
to give the amphiphilic block copolymer according to any one of the claims 1 to 3, which may be optionally further subjected to purification by column chromatography and/or to removal of protecting Boc groups by trifluoroacetic acid and/or to lyophilization.

8. A method of preparation of the polymer conjugate according to any one of the claims 4 or 5, which comprises the following steps:
- a step of radical polymerization of N-(2-hydroxypropyl)methacrylamide, optionally containing up to 20 mol% of the monomer of the general formula (I), where R is aminoacyl selected from the group consisting of glycyl, glycylglycyl, β-alanyl, 6-aminohexanoyl, 4-aminobenzoyl, acyles derived from the oligopeptides of two to five amino acids, especially GlyPheGly, GlyLeuGly, GlyLeuPheGly and GlyPheLeuGly, protected on hydrazide groups by tert-butyloxycarbonyl groups, and/or optionally containing up to 15 mol% of the monomer of the general formula (II), where T is oligopeptide attached to the monomer unit via an amide bond, wherein the oligopeptide is selected from the group consisting of the oligopeptides of two to five amino acids, preferably the oligopeptide is GlyPheGly, GlyLeuGly, GlyLeuPheGly, GlyPheLeuGly, AspProLys , AspProSer and AspProGly; wherein the monomer of the general formula (I) and/or the monomer of the general formula (II) may eventually contain a covalently bound molecule of a low molecular weight drug;
at a temperature ranging from 40 to 100°C, preferably from 50 to 80°C, and a solvent preferably selected from the group comprising DMSO, aqueous buffers, dimethylacetamide, dimethylformamide, methanol, ethanol, dioxane, tetrahydrofuran, propanol, tert-butanol or mixtures thereof, initiated with an initiator, preferably selected from the group comprising ABIK-TT and ABIK-N₃, where ABIK-TT is 2-[(Z)-[1-cyano-1-methyl-4-oxo-4-(2-thioxothiazolidin-3-yl)butyl]azo] -2-methyl-5-oxo-5-(2-thioxothiazolidin-3-yl)pentanenitrile and ABIK-N₃ is N-(3-azidopropyl)-4-[(Z)-[4-(3-azidopropylamino)-1-cyano-1-methyl-4-oxo-butyl]azo]-4-cyano-pentanamide, optionally in the presence of a chain transfer agent, preferably selected from the group of CTA-TT and CTA-N₃, where CTA-TT is [1-cyano-1-methyl-4-oxo-4-(2-thioxothiazolidin-3-yl)butyl]benzencarbodithioate and CTA-N₃ is [4-(3-azidopropylamino)-1-cyano-1-methyl-4-oxo-butyl]benzencarbodithioate, to form the hydrophilic block A, preferably with a terminal TT or N₃ group; optionally containing a covalently bound low molecular weight drug, as a semitelechelic hydrophilic polymer block A, optionally containing a covalently bound low molecular weight drug; wherein the terminal TT or N₃ group of the resulting semitelechelic hydrophilic polymer block A may further react with aminoethylpyridyldisulfide to give a semitelechelic hydrophilic polymer block A with terminal aminoethylpyridyldisulfide (PDS) group, or with a peptide to form a semitelechelic hydrophilic polymer block A with a terminal peptide chain as a precursor of the linker L;
- a step in which the semitelechelic hydrophilic polymer block A from the previous step, eventually containing the covalently bound low molecular weight drug, reacts with the semitelechelic or telechelic hydrophobic polymer block B of general formula Y-PPO-Y, Y-L-PPO-L-Y or PPO-L-Y or 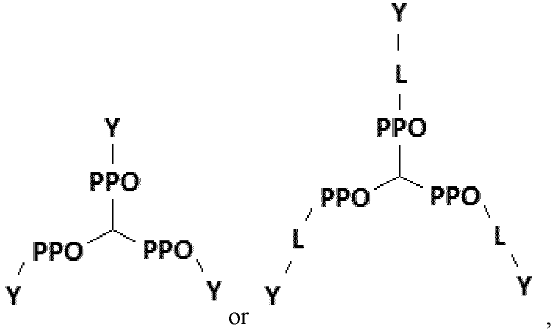 where Y is -NH₂, oligopeptide of two to five amino acids, succinimidyl carbonate or dibenzocyclooctyne group, and where L is peptide with two to five amino acids or -S-S- bond, wherein L may be attached to the block B and group Y by an amide bond or as a part of the linking chain of general formulas (VIII), (IX), (X), (XI),
or with the chain transfer agent PPO-(L-CTA)₂, where L is peptide of two to five amino acids or -S-S- bond,
to give the amphiphilic block copolymer according to any one of the claims 1 to 3, or, optionally, to give the polymer conjugate according to any one of the claims 4 and 5;
- optionally a step of removal of protecting Boc groups;
- optionally a step of conjugation of free hydrazide groups and/or amide groups of monomer units of the general formula (I) or (II) with a low molecular weight drug;
- optionally a step of purification by column chromatography and/or lyophilization.

9. A pharmaceutical composition comprising:
the block copolymer according to the claim 1, 2, 3 or 6 and the low molecular weight drug in its free form, preferably the low molecular weight drug is a low molecular weight cytostatic, most preferably the low molecular weight cytostatic is selected from the group comprising doxorubicin, docetaxel, paclitaxel, pirarubicin, mitomycin C, daunorubicin, larotaxel;
and/or the polymer conjugate according to the claim 4, 5 or 6;
wherein the pharmaceutical composition further comprises at least one pharmaceutically acceptable ingredient selected from the group comprising antiadhesives, binders, coating agents, colouring agents, disintegrants, flavourings, lubricants, preservatives, sweeteners, absorbents.

10. The block copolymer according to the claim 1, 2, 3 or 6 and/or the polymer conjugate according to the claim 4, 5 or 6 and/or the pharmaceutical composition according to the claim 9 for use as a medicament.

11. The block copolymer according to the claim 1, 2, 3 or 6 and/or the polymer conjugate according to the claim 4, 5 or 6 and/or the pharmaceutical composition according to the claim 9 for use as a cytostatic.

12. The block copolymer according to the claim 1, 2, 3 or 6 and/or the polymer conjugate according to the claim 4, 5 or 6 and/or the pharmaceutical composition according to the claim 9 for use as a medicament in the treatment of solid tumours and/or lymphoma and/or leukaemia.

## Patentansprüche

1. Ein Blockcopolymer zur Überwindung der Arzneimittelresistenz von Tumoren gegenüber einer Chemotherapie, umfassend mindestens einen Block A und mindestens einen Block B, wobei mindestens ein Block A und mindestens ein Block B mit einem Linker L miteinander verbunden sind, der hydrolytisch, enzymatisch und/oder reduktiv biologisch abbaubar ist und aus der Gruppe der Oligopeptide mit zwei bis fünf Aminosäuren und/oder eine Disulfidbindung ausgewählt wird, und wobei der hydrophile Block A aus Poly(N-(2-hydroxypropyl)methacrylamid) gebildet wird, das gegebenenfalls bis zu 20 Mol-% Monomereinheiten der allgemeinen Formel (I) enthält, 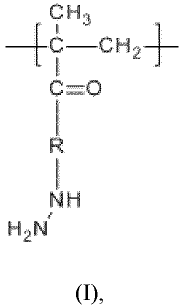
wobei R Aminoacyl ist, ausgewählt aus der Gruppe bestehend aus Glycyl, Glycylglycyl, β-Alanyl, 6-Aminohexanoyl, 4-Aminobenzoyl, Acylen, die von Oligopeptiden mit zwei bis fünf Aminosäuren abgeleitet sind, insbesondere GlyPheGly, GlyLeuGly, GlyLeuPheGly und GlyPheLeuGly; und/oder bis zu 15 Mol-% Monomereinheiten der allgemeinen Formel (II), 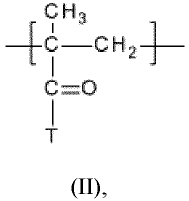
wobei T ein Oligopeptid ist, das über eine Amidbindung an die Monomereinheit gebunden ist, wobei das Oligopeptid aus der Gruppe ausgewählt ist, die aus den Oligopeptiden von zwei bis fünf Aminosäuren besteht, vorzugsweise ist das Oligopeptid GlyPheGly, GlyLeuGly, GlyLeuPheGly, GlyPheLeuGly, AspProLys, AspProSer und AspProGly;
und der hydrophobe Block B wird durch ein Polypropylenoxid gebildet;
wobei das Molekulargewicht *Mₙ* des Blocks A im Bereich von 4.000 bis 60.000 g/mol liegt und das Molekulargewicht *Mₙ* des Blocks B im Bereich von 1.000 bis 17.600 g/mol liegt;
und wobei das Molekulargewicht des Blockcopolymers im Bereich von 5.000 bis 95.000 g/mol liegt;
wobei das Blockcopolymer weiterhin Endgruppen umfassen kann, wobei die Endgruppe von Block A ist 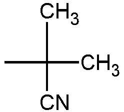 und die Endgruppe von Block B ist ausgewählt aus der Gruppe bestehend aus - NH₂, 2-Aminopropyl, Dibenzocyclooctyl und einem Oligopeptid aus zwei bis fünf Aminosäuren, verbunden mit PPO über eine Amidbindung.

2. Blockcopolymer nach Anspruch 1, **dadurch gekennzeichnet, dass** die Blöcke A und B in einer linearen oder verzweigten Topologie angeordnet sind; vorzugsweise sind die Blöcke A und B in der Reihenfolge A-L-B oder A-L-B-L-A oder B-L-A-L-B oder gemäß einer allgemeinen Formel (III) angeordnet

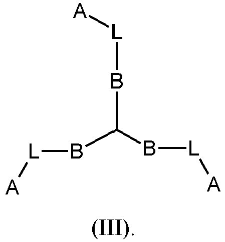

3. Blockcopolymer nach Anspruch 1 oder 2, **dadurch gekennzeichnet, dass** der Linker L ein Oligopeptid ist, ausgewählt aus der Gruppe bestehend aus AspProLys, AspProLysProAsp, GlyLeuGly, GlyPheGly, GlyPheLeuGly, GlyLeuPheGly und/oder eine Disulfidbindung.

4. Ein Polymerkonjugat, das das Blockcopolymer nach Anspruch 1, 2 oder 3 umfasst, das weiter 1 bis 25 Gew.-% eines Arzneimittels mit niedrigem Molekulargewicht enthält, das kovalent an die Monomereinheit der allgemeinen Formel (I) und/oder an die Monomereinheit der allgemeinen Formel (II) des Blocks A durch eine hydrolytisch und/oder enzymatisch spaltbare kovalente Bindung gebunden ist, vorzugsweise ist das Arzneimittel ein niedermolekulares Zytostatikum, am meisten bevorzugt wird das niedermolekulare Zytostatikum aus der Gruppe bestehend aus Doxorubicin, Docetaxel, Paclitaxel, Pirarubicin, Mitomycin C, Daunorubicin, Larotaxel ausgewählt.

5. Polymerkonjugat nach Anspruch 4, **dadurch gekennzeichnet, dass** das Molekül des niedermolekularen Arzneimittels an die Monomereinheit der allgemeinen Formel (I) und/oder an die Monomereinheit der allgemeinen Formel (II) Block A des Polymerkonjugats durch eine Hydrazonoder eine Peptidbindung gebunden ist.

6. Das Blockcopolymer nach einem der Ansprüche 1 bis 3 und/oder das Polymerkonjugat nach einem der Ansprüche 4 und 5, **dadurch gekennzeichnet, dass** es in einem wässrigen Medium in Form einer Mizelle vorliegt, wobei das wässriges Medium umfasst Wasser, Kochsalzlösung, Körperflüssigkeiten, insbesondere Blut und Blutplasma.

7. Verfahren zur Herstellung des Blockcopolymers nach Anspruch 1, 2 oder 3, wobei das Verfahren die folgenden Schritte umfasst:
- einen Schritt der radikalischen Polymerisation von N-(2-Hydroxypropyl)methacrylamid, das gegebenenfalls bis zu 20 Mol-% des Monomers der allgemeinen Formel (I) enthält, wobei R Aminoacyl ist, ausgewählt aus der Gruppe bestehend aus Glycyl, Glycylglycyl, Beta-alanyl, 6-Aminohexanoyl, 4-Aminobenzoyl, Acyle, die von Oligopeptiden mit zwei bis fünf Aminosäuren abgeleitet sind, insbesondere GlyPheGly, GlyLeuGly, GlyLeuPheGly und GlyPheLeuGly, an Hydrazidgruppen durch tert-Butyloxycarbonyl (Boc)-Gruppen geschützt sind, und/oder optional bis zu 15 Mol-% des Monomers der allgemeinen Formel (II) enthält, wobei T ein Oligopeptid ist, das über eine Amidbindung an die Monomereinheit gebunden ist, wobei das Oligopeptid aus der Gruppe bestehend aus Oligopeptiden von zwei bis fünf Aminosäuren ausgewählt ist, vorzugsweise das Oligopeptid ist GlyPheGly, GlyLeuGly, GlyLeuPheGly, GlyPheLeuGly, AspProLys, AspProSer und AspProGly;
bei einer Temperatur im Bereich von 40 bis 100 °C, vorzugsweise von 50 bis 80 °C, und in einem Lösungsmittel, vorzugsweise ausgewählt aus der Gruppe bestehend aus DMSO, wässrigen Puffern, Dimethylacetamid, Dimethylformamid, Methanol, Ethanol, Dioxan, Tetrahydrofuran, Propanol, tert-Butanol oder Mischungen davon, initiiert mit einem Initiator, vorzugsweise ausgewählt aus der Gruppe bestehend aus ABIK-TT und ABIK-N₃, wobei ABIK-TT ist 2-[(Z)-[1-Cyano-1-methyl-4-oxo-4-(2-thioxothiazolidin-3-yl)butyl]azo]-2-methyl-5-oxo-5-(2-thioxothiazolidin-3-yl)pentannitril und ABIK-N₃ ist N-(3-Azidopropyl)-4-[(Z)-[4-(3-azidopropylamino)-1-cyano-1-methyl-4-oxo-butyl]azo]-4-cyano-pentanamid, gegebenenfalls in Gegenwart eines Kettenübertragungsmittels, vorzugsweise ausgewählt aus der Gruppe CTA-TT und CTA-N₃, wobei CTA-TT [1-Cyano-1-methyl-4-oxo-4-(2-thioxothiazolidin-3-yl)butyl]benzolcarbodithioat und CTA-N₃ [ 4-(3-Azidopropylamino)-1-cyano-1-methyl-4-oxo-butyl]benzolcarbodithioat, um den hydrophilen Block A als semitelechelischen hydrophilen Polymerblock A zu bilden, vorzugsweise mit einer terminalen TT- oder N₃-Gruppe; wobei die terminale TT- oder N₃-Gruppe des resultierenden semitelechelischen hydrophilen Polymerblocks A weiter mit Aminoethylpyridyldisulfid reagieren kann, um einen semitelechelischen hydrophilen Polymerblock A mit einer terminalen Aminoethylpyridyldisulfidgruppe (PDS) zu ergeben, oder mit einem Peptid aus zwei bis fünf Aminosäuren, um einen semitelechelischen hydrophilen Polymerblock A mit einer terminalen Peptidkette als Vorläufer des Linkers L zu ergeben;
- einen Schritt, in dem der semitelechelische hydrophile Polymerblock A aus dem vorherigen Schritt reagiert mit dem semitelechelischen oder telechelischen hydrophoben Polymerblock B der allgemeinen Formel Y-PPO-Y, Y-L-PPO-L-Y oder PPO-L-Y oder 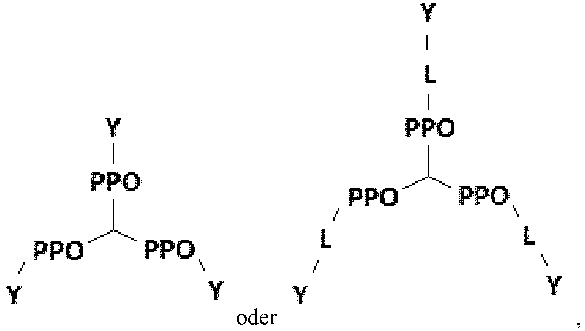 wobei Y ist -NH₂, ein Oligopeptid mit zwei bis fünf Aminosäuren, eine Succinimidylcarbonatoder Dibenzocyclooctingruppe, und wobei L ist ein Oligopeptid mit zwei bis fünf Aminosäuren oder eine -S-S-Bindung, und wobei L an den Block B und die Gruppe Y gebunden sein kann durch eine Amidbindung oder als Teil einer Verbindungskette der 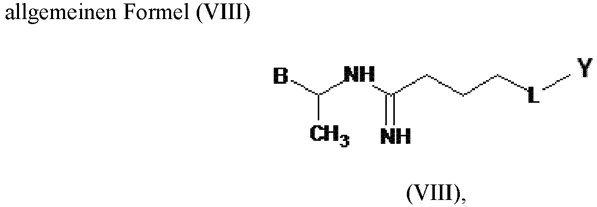 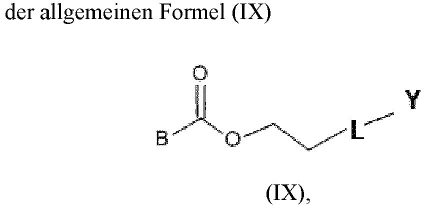 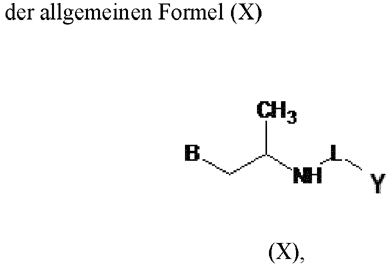 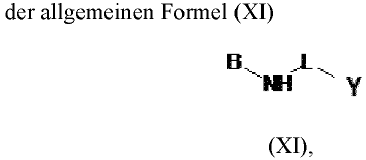 oder mit dem Kettenübertragungsmittel PPO-(L-CTA)₂, wobei L ist ein Peptid aus zwei bis fünf Aminosäuren oder eine -S-S-Bindung,
um das amphiphile Blockcopolymer nach einem der Ansprüche 1 bis 3 zu ergeben, das gegebenenfalls weiter einer Reinigung durch Säulenchromatographie und/oder einer Entfernung von Boc-Schutzgruppen durch Trifluoressigsäure und/oder einer Lyophilisierung unterzogen werden kann.

8. Verfahren zur Herstellung des Polymerkonjugats nach einem der Ansprüche 4 oder 5, das die folgenden Schritte umfasst:
- einen Schritt der radikalischen Polymerisation von N-(2-Hydroxypropyl)methacrylamid, das gegebenenfalls bis zu 20 Mol-% des Monomers der allgemeinen Formel (I) enthält, wobei R ist Aminoacyl, ausgewählt aus der Gruppe bestehend aus Glycyl, Glycylglycyl, β-Alanyl, 6-Aminohexanoyl, 4-Aminobenzoyl, Acyle, die von den Oligopeptiden von zwei bis fünf Aminosäuren abgeleitet sind, insbesondere GlyPheGly, GlyLeuGly, GlyLeuPheGly und GlyPheLeuGly, an Hydrazidgruppen durch tert-Butyloxycarbonylgruppen geschützt sind und/oder gegebenenfalls bis zu 15 Mol % des Monomers der allgemeinen Formel (II) enthält, wobei T ein über eine Amidbindung an die Monomereinheit gebundenes Oligopeptid ist, wobei das Oligopeptid ist ausgewählt aus der Gruppe bestehend aus Oligopeptiden von zwei bis fünf Aminosäuren, vorzugsweise ist das Oligopeptid GlyPheGly, GlyLeuGly, GlyLeuPheGly, GlyPheLeuGly, AspProLys, AspProSer und AspProGly; wobei das Monomer der allgemeinen Formel (I) und/oder das Monomer der allgemeinen Formel (II) gegebenenfalls ein kovalent gebundenes Molekül eines Arzneimittels mit niedrigem Molekulargewicht enthalten kann;
bei einer Temperatur im Bereich von 40 bis 100 °C, vorzugsweise von 50 bis 80 °C, und in einem Lösungsmittel, vorzugsweise ausgewählt aus der Gruppe umfassend DMSO, wässrige Puffer, Dimethylacetamid, Dimethylformamid, Methanol, Ethanol, Dioxan, Tetrahydrofuran, Propanol, tert-Butanol oder Mischungen davon, initiiert mit einem Initiator, vorzugsweise ausgewählt aus der Gruppe bestehend aus ABIK-TT und ABIK-N₃, wobei ABIK-TT ist 2-[(Z)-[1-Cyano-1-methyl-4-oxo-4-(2-thioxothiazolidin-3-yl)butyl]azo]-2-methyl-5-oxo-5-(2-thioxothiazolidin-3-yl)pentannitril und ABIK-N₃ ist N-(3-Azidopropyl)-4-[(Z)-[4-(3-azidopropylamino)-1-cyano-1-methyl-4-oxo-butyl]azo]-4-cyano-pentanamid, gegebenenfalls in Gegenwart eines Kettenübertragungsmittels, vorzugsweise ausgewählt aus der Gruppe bestehend aus CTA-TT und CTA-N₃, wobei CTA-TT ist [1-Cyano-1-methyl-4-oxo-4-(2-thioxothiazolidin-3-yl)butyl]benzolcarbodithioat und CTA-N₃ ist [4-(3-Azidopropylamino)-1-cyano-1-methyl-4-oxo-butyl]benzolcarbodithioat, um den hydrophilen Block A zu bilden, vorzugsweise mit einer terminalen TT- oder N₃-Gruppe; gegebenenfalls einen kovalent gebundenen Wirkstoff mit niedrigem Molekulargewicht enthaltend, als semitelechelischer hydrophiler Polymerblock A, der gegebenenfalls einen kovalent gebundenen Wirkstoff mit niedrigem Molekulargewicht enthält; wobei die terminale TT- oder N₃-Gruppe des resultierenden semitelechelischen hydrophilen Polymerblocks A weiter mit Aminoethylpyridyldisulfid reagieren kann, um einen semitelechelischen hydrophilen Polymerblock A mit terminaler Aminoethylpyridyldisulfid (PDS)-Gruppe zu ergeben, oder mit einem Peptid, um einen semitelechelischen hydrophilen Polymerblock A mit terminaler Peptidkette als Vorläufer des Linkers L zu bilden;
- ein Schritt, in dem der semitelechelische hydrophile Polymerblock A aus dem vorherigen Schritt, der gegebenenfalls den kovalent gebundenen Wirkstoff mit niedrigem Molekulargewicht enthält, reagiert mit dem semitelechelischen oder telechelischen hydrophoben Polymerblock B der allgemeinen Formel Y-PPO-Y, Y-L-PPO-L-Y oder PPO-L-Y oder 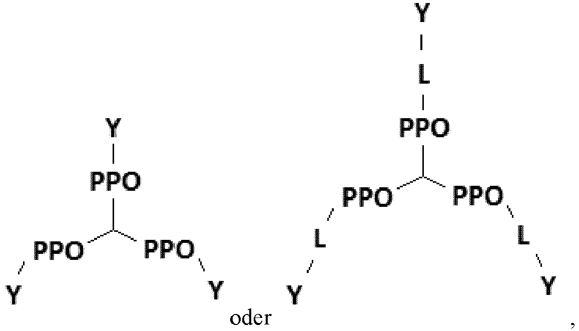 wobei Y ist -NH₂, ein Oligopeptid mit zwei bis fünf Aminosäuren, eine Succinimidylcarbonatoder Dibenzocyclooctingruppe und wobei L ist ein Peptid mit zwei bis fünf Aminosäuren oder eine -S-S-Bindung, wobei L an den Block B und die Gruppe Y gebunden sein kann durch eine Amidbindung oder als Teil der Verbindungskette der allgemeinen Formeln (VIII), (IX), (X), (XI),
oder mit dem Kettenübertragungsmittel PPO-(L-CTA)₂, wobei L ein Peptid aus zwei bis fünf Aminosäuren oder eine -S-S-Bindung ist,
um das amphiphile Blockcopolymer nach einem der Ansprüche 1 bis 3 zu ergeben, oder gegebenenfalls, um das Polymerkonjugat nach einem der Ansprüche 4 und 5 zu ergeben;
- optional einen Schritt zur Entfernung Boc-Schützgruppen;
- optional einen Schritt der Konjugation freier Hydrazidgruppen und/oder Amidgruppen von Monomereinheiten der allgemeinen Formel (I) oder (II) mit einem Arzneimittel mit niedrigem Molekulargewicht;
- optional einen Reinigungsschritt durch Säulenchromatographie und/oder Lyophilisierung.

9. Eine pharmazeutische Zusammensetzung, umfassend:
das Blockcopolymer nach Anspruch 1, 2, 3 oder 6 und das Arzneimittel mit niedrigem Molekulargewicht in seiner freien Form, vorzugsweise ist das Arzneimittel mit niedrigem Molekulargewicht ein Zytostatikum mit niedrigem Molekulargewicht, am meisten bevorzugt wird das Zytostatikum mit niedrigem Molekulargewicht aus der Gruppe umfassend Doxorubicin, Docetaxel, Paclitaxel, Pirarubicin, Mitomycin C, Daunorubicin, Larotaxel ausgewählt;
und/oder das Polymerkonjugat nach Anspruch 4, 5 oder 6;
wobei die pharmazeutische Zusammensetzung weiter mindestens einen pharmazeutisch verträglichen Inhaltsstoff umfasst, der aus der Gruppe ausgewählt ist, die Antihaftmittel, Bindemittel, Beschichtungsmittel, Farbstoffe, Sprengmittel, Geschmacksstoffe, Gleitmittel, Konservierungsmittel, Süßstoffe und Absorptionsmittel umfasst.

10. Das Blockcopolymer nach Anspruch 1, 2, 3 oder 6 und/oder das Polymerkonjugat nach Anspruch 4, 5 oder 6 und/oder die pharmazeutische Zusammensetzung nach Anspruch 9 zur Verwendung als Arzneimittel.

11. Das Blockcopolymer nach Anspruch 1, 2, 3 oder 6 und/oder das Polymerkonjugat nach Anspruch 4, 5 oder 6 und/oder die pharmazeutische Zusammensetzung nach Anspruch 9 zur Verwendung als Zytostatikum.

12. Das Blockcopolymer nach Anspruch 1, 2, 3 oder 6 und/oder das Polymerkonjugat nach Anspruch 4, 5 oder 6 und/oder die pharmazeutische Zusammensetzung nach Anspruch 9 zur Verwendung als Arzneimittel bei der Behandlung von soliden Tumoren und/oder Lymphomen und/oder Leukämie.

## Revendications

1. Un copolymère à blocs pour surmonter la résistance des tumeurs à la chimiothérapie, comprenant au moins un bloc A et au moins un bloc B, dans lequel au moins un bloc A et au moins un bloc B sont reliés l'un à l'autre par un élément de liaison L, qui est biodégradable par voie hydrolytique, enzymatique et/ou réductrice et qui est choisi dans le groupe comprenant des oligopeptides de deux à cinq acides aminés et/ou une liaison disulfure, et dans lequel le bloc hydrophile A est formé par du poly(N-(2-hydroxypropylméthacrylamide)), contenant optionnellement jusqu'à 20 % en moles d'unités monomères de formule générale (I), 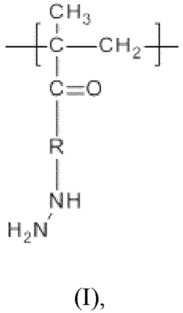
où R est un aminoacyle choisi dans le groupe constitué de glycyl, glycylglycyl, β-alanyl, 6-aminohexanoyl, 4-aminobenzoyl, acyles dérivés d'oligopeptides de deux à cinq acides aminés, en particulier GlyPheGly, GlyLeuGly, GlyLeuPheGly et GlyPheLeuGly ;
et/ou jusqu'à 15 % en moles d'unités monomères de formule générale (II), 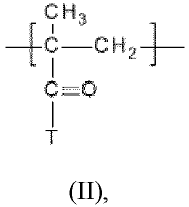
où T est un oligopeptide attaché à l'unité monomère par une liaison amide, l'oligopeptide étant choisi dans le groupe constitué des oligopeptides de deux à cinq acides aminés, de préférence l'oligopeptide est GlyPheGly, GlyLeuGly, GlyLeuPheGly, GlyPheLeuGly, AspProLys, AspProSer, et AspProGly ;
et le bloc hydrophobe B est formé par un oxyde de polypropylène ;
dans lequel le bloc A a un poids moléculaire *Mₙ* dans la plage de valeurs numériques de 4 000 à 60 000 g/mol, et le bloc B a un poids moléculaire *Mₙ* dans la plage de valeurs numériques de 1 000 à 17 600 g/mol ;
et dans lequel le poids moléculaire du copolymère à blocs est dans la plage de valeurs numériques de 5 000 à 95 000 g/mol ;
où le copolymère à blocs peut en outre comprendre des groupes terminaux, le groupe terminal du bloc A étant 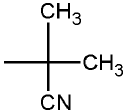 et le groupe terminal du bloc B étant choisis dans le groupe constitué de -NH₂, 2-aminopropyl, dibenzocyclooctyle et un oligopeptide de deux à cinq acides aminés, relié à la PPO par une liaison amide.

2. Le copolymère à blocs selon la revendication 1, **caractérisé en ce que** les blocs A et B sont disposés selon une topologie linéaire ou ramifiée ; de préférence, les blocs A et B sont disposés dans l'ordre A-L-B ou A-L-B-L-A ou B-L-A-L-B, ou selon une formule générale (III)

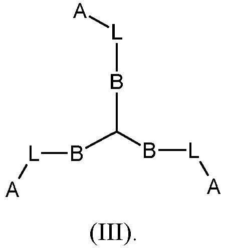

3. Le copolymère à blocs selon la revendication 1 ou 2, **caractérisé par le fait que** l'élément de liaison L est un oligopeptide choisi dans le groupe constitué par AspProLys, AspProLysProAsp, GlyLeuGly, GlyPheGly, GlyPheLeuGly, GlyLeuPheGly et/ou une liaison disulfure.

4. Un polymère conjugué, qui comprend le copolymère à blocs selon la revendication 1, 2 ou 3, qui contient en outre de 1 à 25 % en poids d'un médicament de bas poids moléculaire lié de manière covalente à l'unité monomère de la formule générale (I) et/ou à l'unité monomère de la formule générale (II) du bloc A par une liaison covalente clivable hydrolytiquement et/ou enzymatiquement, de préférence, le médicament est un cytostatique de bas poids moléculaire ; de préférence, le cytostatique de bas poids moléculaire est choisi dans le groupe comprenant la doxorubicine, le docétaxel, le paclitaxel, la pirarubicine, la mitomycine C, la daunorubicine, le larotaxel.

5. Le polymère conjugué selon la revendication 4, **caractérisé en ce que** la molécule du médicament de bas poids moléculaire est liée à l'unité monomère de la formule générale (I) et/ou à l'unité monomère de la formule générale (II) du bloc A du polymère conjugué par une hydrazone ou une liaison peptidique.

6. Le copolymère à blocs selon l'une quelconque des revendications 1 à 3 et/ou le polymère conjugué selon l'une quelconque des revendications 4 et 5, **caractérisé en ce qu'**il est dans un milieu aqueux sous forme de micelle, le milieu aqueux comprenant l'eau, le sérum physiologique, les fluides corporels, en particulier le sang et le plasma sanguin.

7. Une procédé de préparation du copolymère à blocs selon la revendication 1, 2 ou 3, dans laquelle ladite méthode comprend les étapes suivantes :
- une étape de polymérisation radicale du N-(2-hydroxypropyl)méthacrylamide, contenant optionnellement jusqu'à 20 % en moles du monomère de la formule générale (I), où R est un aminoacyle choisi dans le groupe constitué de glycyl, glycylglycyl, bêta-alanyl, 6-aminohexanoyl, 4-aminobenzoyl, acyles dérivés d'oligopeptides de deux à cinq acides aminés, en particulier GlyPheGly, GlyLeuGly, GlyLeuPheGly et GlyPheLeuGly, protégés sur les groupes hydrazides par des groupes tert-butyloxycarbonyl (Boc), et/ou contenant optionnellement jusqu'à 15 % en moles du monomère de la formule générale (II), où T est l'oligopeptide attaché à l'unité monomère par une liaison amide, où l'oligopeptide est choisi dans le groupe qui consiste des oligopeptides de deux à cinq acides aminés, de préférence l'oligopeptide est GlyPheGly, GlyLeuGly, GlyLeuPheGly, GlyPheLeuGly, AspProLys, AspProSer et AspProGly ;
à une température dans la plage de valeurs numériques de 40 à 100 °C, de préférence de 50 à 80 °C, et dans un solvant de préférence choisi dans le groupe comprenant le DMSO, les tampons aqueux, le diméthylacétamide, le diméthylformamide, le méthanol, l'éthanol, le dioxane, le tétrahydrofurane, le propanol, le tert-butanol ou leurs mélanges, initié avec un initiateur, de préférence choisi dans le groupe comprenant ABIK-TT et ABIK-N₃, où ABIK-TT est 2-[(Z)-[1-cyano-1-méthyl-4-oxo- 4-(2-thioxothiazolidin-3-yl)butyl]azo]-2-méthyl-5-oxo-5-(2-thioxothiazolidin-3-yl)pentanenitrile et ABIK-N₃ est N-(3-azidopropyl)-4 -[(Z)-[4-(3-azidopropylamino)-1-cyano-1-méthyl-4-oxo-butyl]azo]-4-cyano-pentanamide, optionnellement en présence d'un agent de transfert de chaîne, de préférence choisi dans le groupe de CTA-TT et CTA-N₃, où CTA-TT est le [1-cyano-1-méthyl-4-oxo-4-(2-thioxothiazolidin-3-yl)butyl]benzencarbodithioate et CTA-N₃ est le [4-(3-azidopropylamino)-1-cyano-1-méthyl-4-oxo-butyl]benzencarbodithioate, pour former le bloc hydrophile A sous la forme d'un bloc polymère hydrophile semitéléchélique A, de préférence avec un groupe terminal TT ou N₃ ; dans lequel le groupe terminal TT ou N₃ du bloc polymère hydrophile semi-téléchélique A résultant peut en outre réagir avec l'aminoéthylpyridyldisulfure pour donner le bloc polymère hydrophile semi-téléchélique A avec un groupe terminal aminoéthylpyridyldisulfure (PDS), ou avec un peptide de deux à cinq acides aminés pour donner un groupe semitéléchélique bloc polymère hydrophile A avec une chaîne peptidique terminale, comme un précurseur du liaison L ;
- une étape dans laquelle le bloc polymère hydrophile semitéléchélique A de l'étape précédente réagit avec le bloc polymère hydrophobe semitéléchélique ou téléchélique B de formule générale Y-PPO-Y, Y-L-PPO-L-Y ou PPO-L-Y ou 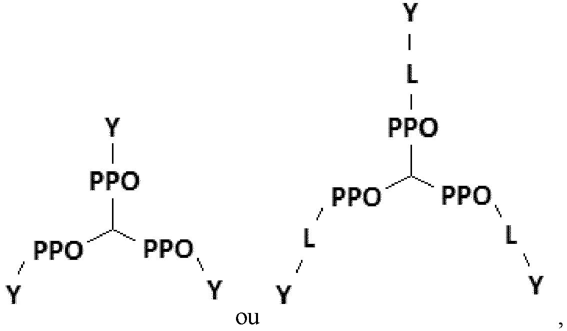 dans laquelle Y est -NH₂, un oligopeptide de deux à cinq acides aminés, un carbonate de succinimidyle ou un groupe dibenzocyclooctyne, et dans lequel L est un oligopeptide de deux à cinq acides aminés ou une liaison -S-S-, et dans lequel L peut être attaché au bloc B et au groupe Y par une liaison amide ou en tant que partie d'une chaîne de liaison de formule générale (VIII) 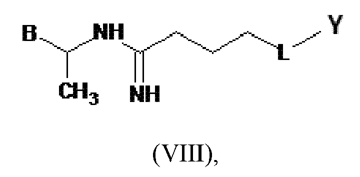 de forme générale (IX) 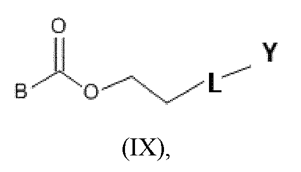 de forme générale (X) 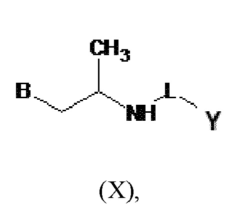 de forme générale (XI) 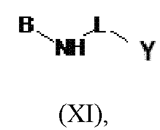 ou avec l'agent de transfert de chaîne PPO-(L-CTA)₂, où L est un peptide de deux à cinq acides aminés ou une liaison -S-S-,
pour donner le copolymère à blocs amphiphile selon l'une quelconque des revendications 1 à 3, qui peut être optionnellement soumis en outre à une purification par chromatographie sur colonne et/ou à l'élimination des groupes Boc protecteurs par l'acide trifluoroacétique et/ou à une lyophilisation.

8. Une procédé de préparation du polymère conjugué selon l'une quelconque des revendications 4 ou 5, qui comprend les étapes suivantes :
- une étape de polymérisation radicalaire du N-(2-hydroxypropyl)méthacrylamide, contenant optionnellement jusqu'à 20 % en moles du monomère de formule générale (I), où R est un aminoacyle choisi dans le groupe constitué par glycyle, glycylglycyle, β- alanyl, 6-aminohexanoyle, 4-aminobenzoyl, acyles dérivés des oligopeptides de deux à cinq acides aminés, notamment GlyPheGly, GlyLeuGly, GlyLeuPheGly et GlyPheLeuGly, protégés sur des groupements hydrazide par des groupements tert-butyloxycarbonyle, et/ou contenant optionnellement jusqu'à 15 % en moles du monomère de formule générale (II), dans laquelle T est un oligopeptide lié à l'unité monomère via une liaison amide, dans laquelle l'oligopeptide est choisi dans le groupe constitué des oligopeptides de deux à cinq acides aminés, de préférence l'oligopeptide est GlyPheGly, GlyLeuGly, GlyLeuPheGly, GlyPheLeuGly, AspProLys, AspProSer et AspProGly ; dans lequel le monomère de formule générale (I) et/ou le monomère de formule générale (II) peuvent optionnellement contenir une molécule liée de manière covalente d'un médicament de bas poids moléculaire ;
à une température dans la plage de valeurs numériques de 40 à 100 °C, de préférence de 50 à 80 °C, et un solvant choisi de préférence dans le groupe comprenant le DMSO, les tampons aqueux, le diméthylacétamide, le diméthylformamide, le méthanol, l'éthanol, le dioxane, le tétrahydrofuranne, le propanol, le tert-butanol ou des mélanges de ceux-ci, initiés avec un initiateur, de préférence choisi dans le groupe comprenant ABIK-TT et ABIK-N₃, où ABIK-TT est 2-[(Z)-[1-cyano-1-méthyl-4-oxo-4- (2-thioxothiazolidin-3-yl)butyl]azo]-2-méthyl-5-oxo-5-(2-thioxothiazolidin-3-yl)pentanenitrile et ABIK-N₃ est N-(3-azidopropyl)-4-[ (Z)-[4-(3-azidopropylamino)-1-cyano-1-méthyl-4-oxo-butyl]azo]-4-cyano-pentanamide, optionnellement en présence d'un agent de transfert de chaîne, de préférence choisi parmi les groupe de CTA-TT et CTA-N₃, où CTA-TT est le [1-cyano-1-méthyl-4-oxo-4-(2-thioxothiazolidin-3-yl)butyl]benzencarbodithioate et CTA-N₃ est le [4-(3-azidopropylamino)-1-cyano-1-méthyl-4-oxo-butyl]benzencarbodithioate, pour former le bloc hydrophile A, de préférence avec un groupe terminal TT ou N₃ ; contenant optionnellement un médicament de bas poids moléculaire lié de manière covalente, sous la forme d'un bloc polymère hydrophile semi-téléchélique A, contenant optionnellement un médicament de bas poids moléculaire lié de manière covalente ; dans lequel le groupe terminal TT ou N₃ du bloc polymère hydrophile semi-téléchélique A résultant peut en outre réagir avec l'aminoéthylpyridyldisulfure pour donner un bloc polymère hydrophile semi-téléchélique A avec un groupe aminoéthylpyridyldisulfure (PDS) terminal, ou avec un peptide pour former un bloc polymère hydrophile semi-téléchélique A avec un chaîne peptidique terminale comme précurseur du liaison L ;
- une étape dans laquelle le bloc polymère hydrophile semitéléchélique A de l'étape précédente, contenant optionnellement le médicament de bas poids moléculaire lié de manière covalente, réagit avec le bloc polymère hydrophobe semitéléchélique ou téléchélique B de formule générale Y-PPO-Y, Y-L-PPO-L-Y ou PPO-L-Y ou 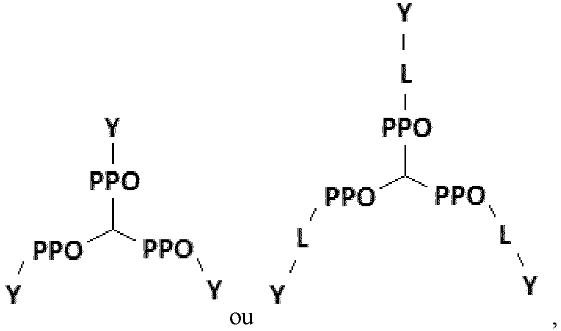 où Y est -NH₂, un oligopeptide de deux à cinq acides aminés, un carbonate de succinimidyle ou un groupe dibenzocyclooctyne, et où L est un peptide de deux à cinq acides aminés ou une liaison -S-S-, où L peut être attaché au bloc B et au groupe Y par une liaison amide ou comme partie de la chaîne de liaison des formules générales (VIII), (IX), (X), (XI),
ou avec l'agent de transfert de chaîne PPO-(L-CTA)₂, où L est un peptide de deux à cinq acides aminés ou une liaison -S-S-,
pour donner le copolymère à blocs amphiphile selon l'une quelconque des revendications 1 à 3, ou, optionnellement, pour donner le polymère conjugué selon l'une quelconque des revendications 4 et 5 ;
- optionnellement, une étape d'élimination des groupes Boc protecteurs ;
- optionnellement, une étape de conjugaison de groupes hydrazide libres et/ou de groupes amide d'unités monomères de formule générale (I) ou (II) avec un médicament de bas poids moléculaire ;
- optionnellement, une étape de purification par chromatographie sur colonne et/ou lyophilisation.

9. Une composition pharmaceutique comprenant :
le copolymère à blocs selon la revendication 1, 2, 3 ou 6 et le médicament de bas poids moléculaire sous sa forme libre, de préférence le médicament de bas poids moléculaire est un cytostatique de bas poids moléculaire, de préférence le cytostatique de bas poids moléculaire est choisi dans le groupe comprenant la doxorubicine, le docétaxel, le paclitaxel, la pirarubicine, la mitomycine C, la daunorubicine, le larotaxel ;
et/ou le polymère conjugué selon la revendication 4, 5 ou 6 ;
où la composition pharmaceutique comprenant en outre au moins un ingrédient pharmaceutiquement acceptable choisi dans le groupe comprenant les antiadhésifs, les liants, les agents d'enrobage, les colorants, les désintégrants, les arômes, les lubrifiants, les conservateurs, les édulcorants et les absorbants.

10. Le copolymère à blocs selon la revendication 1, 2, 3 ou 6 et/ou le polymère conjugué selon la revendication 4, 5 ou 6 et/ou la composition pharmaceutique selon la revendication 9 pour utilisation comme médicament.

11. Le copolymère à blocs selon la revendication 1, 2, 3 ou 6 et/ou le polymère conjugué selon la revendication 4, 5 ou 6 et/ou la composition pharmaceutique selon la revendication 9 pour utilisation comme cytostatique.

12. Le copolymère à blocs selon la revendication 1, 2, 3 ou 6 et/ou polymère conjugué selon la revendication 4, 5 ou 6 et/ou composition pharmaceutique selon la revendication 9 pour utilisation comme médicament dans le traitement de tumeurs solides et/ou de lymphomes et/ou de leucémies.
